# EUROPEAN PATENT SPECIFICATION

(11) **EP 1 007 937 B1**
(45) Date of publication and mention of the grant of the patent: **29.10.2003**
(21) Application number: 98943358.6
(22) Date of filing: 25.08.1998
(51) Int. Cl.: G01N 1/31

(54) **METHOD AND APPARATUS FOR AUTOMATICALLY FORMING MONOLAYERS FROM PARTICULATE MATTER SEPARATED FROM FLUID SAMPLES**
VORRICHTUNG UND VERFAHREN ZUR AUTOMATISCHEN GESTALTUNG VON MONO-SCHICHTEN AUS VON FLÜSSIGEN PROBEN ABGESCHIEDENEN TEILCHEN
PROCEDE ET APPAREIL DE FORMATION AUTOMATIQUE DE COUCHES MONOMOLECULAIRES A PARTIR D'UNE MATIERE PARTICULAIRE SEPAREE D'ECHANTILLONS FLUIDES

(30) Priority: 25.08.1997 US 56445 P; 01.04.1998 US 53010
(43) Date of publication of application: 14.06.2000
(73) Proprietor: MonoGen, Inc., Vernon Hills, IL 60061 (US)
(72) Inventor: GUIRGUIS, Raouf, A., Vienna, VA 22182 (US); YORK, Frederick, J., Longwood, FL 32779 (US); STAFFORD, Roland, T., Ormond Beach, FL 32174-4890 (US); CHRISTENSEN, John, M., DeLand, FL 32720 (US)
(74) Representative: Evens, Paul Jonathan
(86) International application number: PCT/US98/17524
(87) International publication number: WO 99/010723

(56) References cited:
- EP-A- 0 740 142
- EP-A- 0 743 524
- US-A- 5 143 627
- US-A- 5 441 699
- US-A- 5 471 994

## Description

### BACKGROUND OF THE INVENTION

### Field of the Invention

The present invention is directed to apparatuses and methods for collecting a uniform monolayer of particulate matter. In particular, the present invention is directed to semi-automated or automated apparatuses and methods for collecting a uniform monolayer of cells from body fluids and preparing the monolayer of cells for use in cytological protocols.

### Description of Related Art

In a wide variety of technologies, the ability and/or facility for separating matter, typically particulate matter, from a fluid is a critical component in the ability to test for the presence of substances in the fluid. Too often, interference associated with sample preparation obscures the target cells to such a degree that the process is not sufficiently reliable, or too costly.

Such a scenario applies to many other fields which involve detection and/or diagnosis, including environmental testing, radiation research, cancer screening, cytological examination, microbiological testing, and hazardous waste contamination, to name just a few.

In the case of cytological examination, a sample of cells is obtained from a patient. Typically, this is done by scraping or swabbing an area, as in the case of cervical samples, or by collecting body fluids, such as those obtained from the chest cavity, bladder, or spinal canal, or by fine needle aspiration. In a conventional manual cytological examination, particulate matter including cells and debris in the fluid are transferred onto a glass slide by smearing and subsequently air-dried. Smearing results in non-uniform densities and uneven distributions of cells and debris that often obscure the target cells. Air drying causes cell distortion and further impedes accurate examination. In a conventional automated cytological examination, the sample must be spun in a centerfuge to concentrate the cells, the superlatent must be drawn off, and the cells must then be mixed into a carrier fluid. This process is time consuming, often requiring 30 minutes or more per sample, and requires the transfer of the target cells to several containers that must either be cleaned or discarded for each sample, thus increasing the likelihood of contamination. Moreover, an experienced practitioner is required to subjectively evaluate the resulting liquid suspension and decide if the process needs to be repeated. A filter assembly is then placed in the liquid suspension to disperse and capture the cells on the filter, again increasing the risk of contamination. The filter is then removed and placed in contact with a microscope slide for viewing.

In all of these endeavors, limiting factors in the sample preparation protocol include adequately separating particulate matter from its fluid carrier (e.g., physiological fluid, biological fluid and environmental fluid), and easily and efficiently collecting and concentrating the particulate matter in a form readily accessible for microscopic examination.

The prior art contains a number of methods, apparatuses, and structures for dispersing cells in the fluid. For example, U.S. Patent 5,143,627 opens the sample container, inserts a dispersing element into the liquid suspension, and rotates the dispersing element for several minutes. In another example of the prior art, the Saccomanno method is used to process sputum, a process that is time consuming and involves a large number of processing steps.

It has been found that prompt processing of urine to obtain fresh cells ensures the accuracy of quantitative culture results, urinalysis and microscopy. Fresh cells tend to stick to a glass slide much better than cells from preserved urine, allowing for smoother cell spread onto the glass body. Delays in processing, negligent care in either inpatient or outpatient settings and lack of refrigeration may lead to non-optimal slide preparation. One known solution to the delay problem is the use of chemical preservatives with the urine. The presence of liquid preservatives, however, in the urine specimen raises the specific gravity of the specimen to unmeasurable levels and may limit the potential usefulness of the urine for various types of traditional quantitative analysis, such as slide microscopy.

Diagnostic microbiology and/or cytology, particularly in the area of clinical pathology, bases diagnoses on a microscopic examination of cells and other microscopic analyses. The accuracy of the diagnosis and the preparation of optimally interpretable specimens typically depends upon adequate sample preparation. New methodologies such as immunocytochemistry and image analysis require preparations that are reproducible, fast, biohazard-free and inexpensive. Conventional cell preparation techniques fail to adequately address the issues of non-uniform cell densities, uneven cell distribution and air drying artifacts.

A number of urine or other biological fluid specimen containers have been developed to allow liquid biological specimens to be tested without removing the lid of the urine or biological fluid container. None of the prior art solves the problem of transferring cells in a uniform layer to a slide for examination while at the same time preserving the fluid from which the cells were taken.

Conventionally, body fluid samples are collected for cytological examinations using containers that contain a preservative solution for preserving the cytology specimen during shipment from the collection site to the cytology laboratory. Furthermore, cytology specimens collected from the body cavities using a swab, smear, flush or brush are also preserved in containers with fixatives (e.g., alcohol or acetone fixatives) prior to transferring cells onto the slide or membrane for staining or examination.

It is desirable to provide a urine or other biological fluid specimen container that would allow liquid biological specimens to be tested without removing the lid of the urine or biological fluid container. However, none of the prior art solves the problems of transferring cells in a monolayer to a slide for examination without submerging portions of the device in the sample (and increasing the risk of contamination), consistently and repeatedly forming a high quality monolayer on the microscope slide, and processing the sample so that the fluid from which the cells were taken is preserved.

Another limiting factor in optimally preparing the particulate matter for microscopic examination involves the solution and/or solutions for fixing the particulate matter to a microscope slide or the like.

Cytologic specimens, which constitute the examinable form of the cytologic material, may be prepared by well-understood smear or fluid techniques. Because there may be a considerable lapse of time before these specimens are further processed by staining, applying a cover slip, and so forth, however, it is important to apply a fixative to the cytologic material as a means of preserving and fixing the cells.

Properly fixing (i.e., preserving) cytologic material such as cells, cell aggregates and small tissue fragments derived from cytologic collections of human or animal tissue is a prerequisite to the accurate diagnosis of disease, especially cancer. Cytologic material must be fixed as soon as possible after obtaining the material to prevent cell distortion.

Air-dried and tetrachrome-dye stained cytologic specimens, although popular abroad, are not generally used in the United States. Rather, wet fixation, either by the immersion of slides into an alcohol solution, by saturation of slides with a spray fixative or by directly discharging cytologic material into an alcohol solution, is a known method of cell fixation. Cell fixation is a prerequisite for interpretable Papanicolaou, Hematoxylin and Eosin or other stained cytologic specimen slides.

Generally, alcohol solutions, with or without other additives such as polyethylene glycol, ranging from 50% to 95% (v/v: methanol, ethanol, isopropanol) are known solutions for use in wet fixation. When alcohol solutions greater than 50% (v/v) are used for collecting and fixing fluids high in protein, however, a protein sediment forms which subsequently hardens. Protein sedimentation makes the fixed cytologic material difficult to transfer to glass slides for examination, regardless of whether the transfer is done by direct application to the glass slide, by cytofiltration through a small pore filter, or by cytocentrifugtion onto glass slides coated with an adhesive such as chrome aluminum gelatin.

For over a century, tissue fixative compositions used to preserve and prepare tissue for analytical evaluation have been based on formaldehyde. The standard composition employed for tissue preservation and the preparation of thin-cut tissue for microscopic examination is Formalin. Formalin is a 3 to 10 percent solution of formaldehyde in water, usually containing about 15 percent methyl alcohol. Alcohol improves the preservative properties of the solution. Despite numerous disadvantages, most notably high toxicity and irritant properties, Formalin remains the fixative of choice in typical laboratory applications owing to its rapid reaction with exposed tissue surfaces and consequent maximized cellular preservation. Methanol may adversely affect the texture of the tissue, rendering it too brittle or, more usually, too soft for ease in cutting for slide preparation. It also may produce pigmented artifacts or impurities that interfere with staining. Formalin containing methanol nevertheless provides preserved tissue that can be satisfactorily sectioned and stained for microscopic examination.

Histologists have long endeavored to develop effective immunohistochemical fixatives and morphologic fixatives. Moreover it is desirable to preserve morphologic detail preserve tissue antigens to permit immunohistochemical detection and localization of antigens in tissue.

Such fixatives render protein insoluble. For example, formaldehyde may be used as a crosslinking agent forming covalent bonds between the aldehyde groups and specific amino acids to stabilize protein structure and transform the cell cytoplasm into a gel which prohibits movement of autolytic enzymes. Alternately, alcohol may be used as a fixative to precipitate protein through denaturation.

Preferably, a fixative should retard autolysis and putrefaction and preserve morphologic detail and antigenicity. Unfortunately, an effective morphologic fixative is not necessarily an effective immunohistochemical fixative.

In contrast to the conventional techniques, the solid matter preparation techniques of the present invention address the issues of non-uniform matter densities, uneven matter distribution, and sample loss and contamination due to the number of steps involved in the sample preparation. Thus, preparations according to the present invention result in an even distribution of solids that have superior morphology, improved visualization, and are readily positioned and available for light absorbance analysis without the need to further manipulate or prepare the sample.

### SUMMARY OF THE INVENTION

The present invention relates to apparatuses and methods for collecting matter for detection, analysis, quantification, and/or visualization. The automated devices and methods of the present invention are particularly suitable for separating matter from biological, physiological, and environmental fluids and presenting the particulate matter in an improved manner for cytological examination.

Aspects of the present invention are defined in the appendent independent claims, to which reference should now be made. Furthermore, embodiments of the invention are defined in the appendent dependent claims, to which reference should also now be made.

The present invention relates to semi-automated and automated apparatuses and methods for collecting a uniform layer of particulate matter from a fluid specimen in a collection apparatus or assay module, and for transferring the uniform layer of particulate matter to a slide. Such an apparatus according to the present invention overcomes problems associated with conventional equipment for collecting cells and other particles for cytology by providing a mechanism of relatively simple structure and operation that separates particles from a liquid solution, collects an approximately known quantity of the cells in a monolayer, and transfers the collected cells to a microscope slide. In some embodiments of the present invention, no element of the apparatus is placed in the liquid sample, thus preventing unnecessary contamination of the sample. Moreover, in some embodiments of the present invention, the container holding the sample is not opened in the course of collecting and transferring the cells, thus eliminating the possibility of sample contamination by the apparatus. In all embodiments of the present invention, a monolayer of the particulate matter, e.g., cells, in the sample is collected on a filter by passing two branches of a fluid flow through and around the filler. Such a filter is known from U.S. Patent Numbers 5,139,031, 5,301,685 and 5,471,994.

According to one embodiment of the present invention, the collection of a monolayer of cells for cytological examination allows a uniform cell slide to be obtained without contamination of the cells by preservatives, workers or outside materials. The transfer of cells from a sample container to the cytology collection apparatus may be carried out without pouring or pipetting the collected specimen.

The present invention is also directed to a cell collection container system that can be easily disassembled to allow face-to-face transfer of cells from the device to a slide for microscope examination. The cell collection container according to one embodiment of the present invention provides an apparatus and method for collecting a monolayer of cells that can be transferred to a microscope slide.

The apparatuses and methods according to the present invention obviate the need for a trained technician to properly prepare a sample substrate. Thus, time, expense and expertise are eliminated or reduced as critical factors in sample preparation protocols.

The apparatuses and methods of the present invention also provide advantages in sample preparation because they are suitable for use with fresh, untreated cells, unmodified cells, and are particularly designed to provide a thin, uniform layer of solid matter (up to approximately 40 microns or more). One embodiment of the present invention is particularly useful for collecting cells for a Pap smear.

According to another feature of the present invention, the matter collection apparatuses may also include additional modules, removable or integrated, for treating the sample fluid. For example, the sample fluid may be treated with a matter collection module, in combination with a debris removal module, a chromatography module, an assay module, or combinations of these and other devices. These and other modules or treatment protocols provide features that may be desirable to incorporate into a sample preparation apparatus according to the present invention. Examples of sitable devices include those disclosed in U.S. Patents 4,953,561; 5,224,489; 5,016,644; 5,139,031; 5,301,685; 5,042,502; and 5,137,031.

For example, the apparatuses and methods of the present invention have many advantages for conventional microbiology and hematology. The collected cells are in a predetermined area that is easily accessible to a radiant light source and to a wavelength absorbance meter. Because cells are concentrated in a single layer, they are almost always in one focal plane, thus eliminating or reducing interference by other particles and virtually eliminating technician time and expertise in establishing a proper reading. The minimal matter overlap achieved by the present invention ensures that all matter can be easily examined with little chance for critical solids to be obscured by clumps of overlapping solids or debris. Certain embodiments of the apparatuses of the present invention may be used in combination with other automated devices to detect and analyze any solid matter in a given population. They also permit a detailed analysis of the chemical composition of the matter.

In tests using the present invention, transferring the monolayer cells from the filter to a microscope slide has proven to be extremely effective without differential cell loss. Microscopic examination shows that the cell distribution is substantially the same on the slide as on the filter.

An automatic apparatus according to certain embodiments of the present invention concurrently processes a plurality of sample containers mounted on a common transport. In preferred embodiments of the present invention, the covering on the sample container may include a hollow tube with or without a rotatable dispersing element. The present invention agitates the sample within the container to ensure break-up of large particulate matter, e.g., mucoid bodies in the case of sputem samples, and the even distribution of cells throughout the fluid. Agitation may occur as the result of relative motion between components of the sample container, non-uniform motion of the sample container, and/or inertial reaction forces applied to the sample by the container.

According to some embodiments of the present invention, the samples, their containers and filters are subjected to a number of different motions including relative rotation of a dispersing element with respect to the sample fluid. Additionally, these embodiments of the present invention may transport the containers to and from a cell transfer processing stage, such as by rotary and/or translation motion, and may also provide further motions for removing the filter assembly and positioning the filter assembly in contact with a microscope slide assembly. In one embodiment of the invention, the automated apparatus includes a platform having a plurality of filter assemblies, a platform for positioning a plurality of specimen containers, a platform for positioning a plurality of microscope slides and/or filters, a filter loader adjacent to the filter assembly platform, a microscope slide loader adjacent to the microscope slide platform, a microscope slide unloader adjacent to the microscope slide platform, and a control system for operating, monitoring, and sequencing the various assemblies.

The control system monitors the particulate matter collecting operation by monitoring parameters of the liquid flow to determine when a pre-determined quantity of particulate is collected on the filter. An upper assembly of the instrument positions the filter device, with the collected cells on the filter surface, for abutment against a microscope slide.

It is important to the method and apparatus of the invention that the cells maintain the monolayer distribution with which they were collected on the filter as the cells are transferred from the filter device to the microscope slide. The invention thus provides cell collection and transfer means that produce a monolayer of cells on the microscope slide.

An instrument according to the present invention preferably employs a fresh sample vial, an unused filter assembly, and an unused microscope slide for each individual cell specimen. Moreover, the relatively simple operation, and the multiple functions that the instrument performs, minimize the requirements for operator attendance and time, as well as minimizing maintenance and preparation.

An embodiment of the invention includes a movable sample container platform, a sample container having a cap adapted to matingly engage a filter assembly, a movable filter assembly platform, one or more microscope slide loader/unloader assemblies adapted to engage the filter, and a microscope slide positioned on the microscope slide loader/unloader assembly. One embodiment of the invention includes multiple iterations of each of the subassemblies described above, so that a preferred automated apparatus according to the invention can process at least two, typically five or more, specimens at the same time or sequentially.

Additional objects and advantages of the invention will be set forth in the description that follows, and in part will be obvious from the description, or may be learned by practice of the invention. The objects and advantages of the invention may be realized and obtained by means of the instrumentalities and combinations particularly pointed out in the appended claims.

### BRIEF DESCRIPTION OF THE DRAWINGS

The accompanying drawings, which are incorporated in and constitute a part of the specification, illustrate a presently preferred embodiment of the invention, and, together with the general description given above and the detailed description of the preferred embodiment given below, serve to explain the principles of the invention.
Figure 1 is an exploded side view of the filter assembly according to an embodiment of the invention.
Figure 2 is a side view of the filter assembly in its closed position according to an embodiment of the invention.
Figure 3 is a side view of an exemplary specimen container, including a stirring mechanism, according to an embodiment of the invention.
Figure 4 is a top view of a detail of the filter assembly according to an embodiment of the invention.
Figure 5 is a bottom view of a detail of the filter assembly according to an embodiment of the invention.
Figure 6 is a top view of a first exemplary embodiment of the invention.
Figure 7 is a side view of the first exemplary embodiment illustrated in Figure 6.
Figure 8 is a top view of a specimen container holder assembly according to the first embodiment of the invention illustrated in Figure 6.
Figure 9 is a side view of the assembly illustrated in Figure 8.
Figure 10 is a top view of a microscope slide turntable assembly according to the first embodiment of the present invention illustrated in Figure 6.
Figure 11 is a side view of the assembly illustrated in Figure 10.
Figure 12 is a top view of a filter loader assembly according to the first embodiment of the present invention illustrated in Figure 6.
Figure 13 is a side view of the assembly illustrated in Figure 12.
Figure 14a is a top view of a microscope slide unloader assembly according to the first embodiment of the invention illustrated in Figure 6.
Figure 14b is a side view of the assembly illustrated in Figure 14a.
Figure 15 is an exploded side view of a detail of the filter assembly and a specimen container according to the first embodiment of the invention illustrated in Figure 6.
Figure 16 is a top view of a microscope slide unloader assembly according to the first embodiment of the invention illustrated in Figure 6.
Figure 17 is a side view of the assembly illustrated in Figure 16.
Figure 18 is a top view of a stirring assembly according to the first embodiment of the invention illustrated in Figure 6.
Figure 19 is a perspective view of a second exemplary embodiment of the invention
Figure 20 is a front elevation view of the second embodiment illustrated in Figure 19.
Figure 21 is a right side view of the second embodiment illustrated in Figure 19.
Figure 22 is a left side view of the second embodiment illustrated in Figure 19.
Figure 23 is a rear elevation view of the second embodiment illustrated in Figure 19.
Figure 24 is a top plan view of the second embodiment illustrated in Figure 19.
Figure 25 is a perspective detail view of a container support according to the second embodiment illustrated in Figure 19.
Figure 26 is a perspective detail view of a first conveyer according to the second embodiment illustrated in Figure 19.
Figure 27 is front elevation detail view of a sampling station according to the second embodiment illustrated in Figure 19.
Figure 28 is a cross-section view of a detail of the sampling station illustrated in Figure 27.
Figure 29 is a top plan detail view of a slide loader according to the second embodiment illustrated in Figure 19.
Figure 30 is a perspective detail view of the slide loader illustrated in Figure 29.
Figure 31 is a see-through detail view of a blotter according to the second embodiment illustrated in Figure 19.
Figure 32A is a schematic illustration showing a group of containers advanced to the sampling station according to the second embodiment illustrated in Figure 19.
Figure 32B is a schematic illustration showing a group of sampling heads in fluid communication with corresponding containers at the sampling station according to the second embodiment illustrated in Figure 19.
Figure 32C is a schematic illustration showing monolayers of particulate matter from respective samples being transferred to a group of slides corresponding to respective heads according to the second embodiment illustrated in Figure 19.
Figure 33 is a schematic illustration of an exemplary fluid management system according to the first embodiment illustrated in Figure 6.
Figure 34 is a schematic illustration of an exemplary fluid management system according to the second embodiment illustrated in Figure 19.
Figure 35 is a schematic detail illustration of the exemplary fluid management system illustrated in Figure 34.
Figure 36 is a schematic detail illustration of the exemplary fluid management system according to the second embodiment illustrated in Figure 19.
Figure 37 is a schematic illustration of an exemplary control system according to the present invention.
Figure 38 is a work flow diagram according to an exemplary embodiment of the present invention.
Figure 39 is a cross-section view of a third exemplary embodiment of the invention.
Figure 40 is a top plan detail view of the third embodiment illustrated in Figure 39.
Figure 41 is a top plan detail view of the third embodiment illustrated in Figure 39.

### DETAILED DESCRIPTION

An apparatus according to the present invention is an automated collection of assemblies or mechanisms for batch processing samples. The apparatus according to the present invention is particularly useful for removing particulate matter from a liquid and transferring the particulate matter to a microscope slide or other cytological examination element. During operation of the automated apparatus, processing of the liquid, particulate matter, or the sample container containing the sample may include or involve one or more of the following stages or steps: opening the container used to ship or transport the sample to the site of the automated apparatus; attaching a sample container cover that extends into the sample; positioning a filter assembly with respect to the cover for fluid communication with the sample; withdrawing at least a portion of the sample from the container through the filter assembly whereby a portion of the particulate matter contained in the sample is adhered to a membrane in the filter assembly; providing a microscope slide movable to a position adjacent to, aligned with, and/or resting against a portion of the filter assembly. Additionally, mechanisms and/or sub-assemblies may further include: one or more sub-assemblies for replacing a used microscope slide with an unused microscope slide; one or more filter assembly loaders; one or more filter assembly unloaders; one or more microscope slide loaders; one or more microscope slide unloaders; one or more bar code readers; one or more bar code printers; one or more transport mechanisms for moving and/or positioning one of the structures noted above; one or more supports for retaining, positioning, or moving one or more of the structures noted above; one or more motors for moving or positioning one or more of the structures noted above; and one or more control systems for operating, preferably selectively and/or sequentially, one or more of the various structures noted above.

The present invention also involves a method for processing a liquid containing particulate matter, e.g., cells, using an automated apparatus configured according to the invention. The present invention also involves removing particulate matter from a liquid and collecting the particulate matter on a medium suitable for cytological examination of the particulate matter.

The present invention also includes automated devices and methods for collecting fluids, such as biological, physiological, or environmental fluids, removing particulate matter from the fluid, without centrifugation, and diagnosing and testing the matter.

As used herein, "sample" refers to any fluid in combination with solid matter, such as particulate matter, and from which it may be desirable to collect the particulate component from the sample for the purpose of establishing its identity or presence in the sample. Typically, the fluid component of the sample will be a liquid. However, the fluid may also be air or gas. As an example, it may be desirable to determine the presence of cancer cells or certain proteins in the biological fluid, such as urine. In another example, it may be desirable to evaluate the nature of contaminants, such as molecular contaminants, in ultra-pure water used in the electronics industry. Other exemplary fluids include but are not limited to body fluids, such as blood, spinal fluid, or amniotic fluid; bronchial lavage; sputum; fine needle aspirates; ground water; industrial processing fluids; and electronic or medical dialysis fluids, to identify just a few. It is intended that the type of fluid being processed should not limit the invention.

As used herein, "particulate matter" refers to any substance in a fluid that is capable of collection and evaluation, preferably by cytological examination. Exemplary matter includes, but is not limited to cells or cell fragments, proteins, molecules, polymers, rubbers, stabilizers, antioxidants, accelerators, silicones, alkyds, thiokols, paraffins, thermoplastics, bacteria, pesticides, and herbicides. Specific exemplary polymeric matter includes, but is not limited to polyethylene, polypropylene, polyisobutylene, polyacrylonitrile, polyethylene glycol, polyvinylchloride, polystyrene, polysulfide, polymethylmethacrylates, polyethyleneterephthalates, bisphenol A (a common environmental contaminant), ethyl cellulose, nitrocellulose, polyurethane, and nylon. Specific exemplary biological matter includes cancer cells, including distinguishing between metastatic and normal cancer cells; proteins, nucleic acids, antibodies, or the like. It is intended that the type of matter being processed should not limit the invention.

As used herein, "adapted for communication", "communicating", or similar terms refer to any means, structures, or methods for establishing fluid flow through the system, as are well known by practitioners in the art. Exemplary structures are shown in the Figures. For example, a conduit may have a connector adapted to receive or connect to a mated connector on another conduit. As used herein, connector refers to any structure used to form a joint or to join itself to another piece. These connectors or connections establish a fluid flow path through various elements of the apparatus, assembly, or system. Typical connections include but are not limited to mating connections, such as Luer-type, screw-type, friction-type, or connectors that are bonded together.

As used herein, "adapted for engaging", "engagement", "engaging", or similar terms refers to complementary structures that may align, mesh, mate, or rest near, against, or within each other. Exemplary structures include the connectors described above.

As used herein, "batch processing" refers to an operation or operations that are capable of being performed independently and simultaneously on more than one sample without cross-contamination between the samples.

As used herein, "group" refers to a quantity of examples of a feature that are identically and concurrently acted upon or utilized in the course of batch processing. A partial group refers to at least one, but less than a maximum finite number of example of the feature, and a full group refers to the maximum finite number of examples of the feature.

### Sample Container and Cover

In accordance with the invention, a sample is collected using conventional techniques, e.g., by collecting urine or other biological fluid in a specimen container, or by placing a swab or brush in a suitable fluid in the specimen container (as is typical for a Pap smear). In one embodiment of the invention, the specimen or sample is collected in a sample container having the design and function as described below. The sample container is typically covered, and has a portion that may be suitable for engaging a filter assembly as will be described below.

In embodiments of the invention, a specimen cup includes a chamber for collecting a liquid specimen and a cover that establishes fluid communication between the chamber and a filter assembly for separating particulate matter from the fluid and collecting the particulate matter at a collection site. The separated particulate matter is collected in a monolayer on a membrane according to the invention. Other embodiments of the invention also include a cover having a hollow tube establishing fluid communication between the sample and the filter assembly. More preferably, the hollow tube includes means for mixing the specimen and/or dispersing the particulate matter in the specimen.

In accordance with embodiments of the invention, a specimen container 20 includes any container suitable for holding a fluid, preferably a biological fluid. The container 20 includes sidewalls 21 and bottom wall 22 that, in combination, provide a chamber 23 having an open end 24, for collecting, holding, or storing a fluid. Typical fluids include, but are not limited to biological fluids, such as body fluids, wastewater fluids, or the like. Typical body fluids include urine or other biological fluids, such as blood, cerebrospinal fluid (CSF), bronchial lavage, sputum or fine needle aspirates.

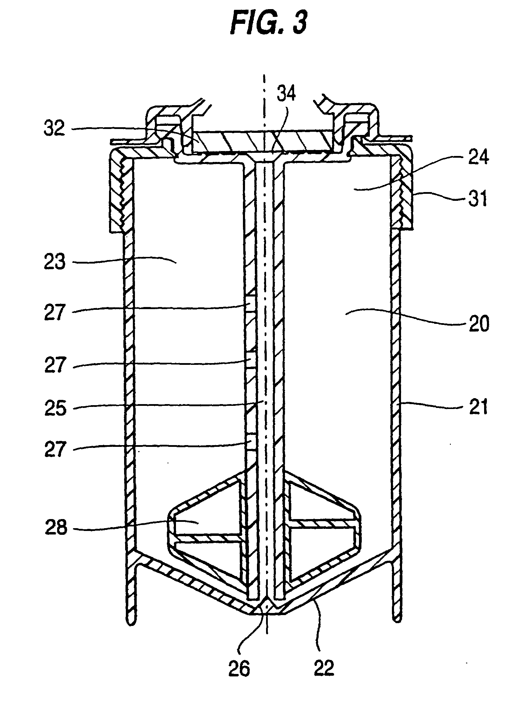

The configuration and materials used to make the cup can be any of a variety of materials, shapes, and sizes. For example, the cup can be constructed of any material compatible with the fluid to be processed. It will be appreciated that the container and the assembly of the sidewalls to the bottom wall can be any conventional assembly. In certain embodiments of the invention, bottom wall 22 is a conical member, as shown in Figure 3. Optionally, bottom wall 22 or side wall 21 may include one or more fins or the like (not shown) extending into the chamber. Such fins may be desirable an embodiment of the invention, described in more detail below, in which the sample in the container is mixed by rotation of the container.

In one embodiment of the invention, the container cover includes a central recessed portion adapted to receive the filter assembly. In some embodiments of the invention, the central recessed portion also communicates with or engages a hollow tube that extends into the specimen container. Optionally, a portion of the tube may include a stirring or dispersing element.

As shown in Figure 3, specimen container 20 includes a tube 25 or the like for drawing the sample from within the container 20. Preferably, tube 25 will be hollow and open or openable at both ends. Tube 25 as illustrated includes open end 26 near the bottom of the container 20, and may include one or more apertures 27 into tube 25 . Open end 26 and/or apertures 27 permit different fluid layers as well as particulate matter and sediments to be simultaneously tested when the sample is withdrawn from the collection chamber 23.

In other preferred embodiments of the invention, an adapter fitting is interposed between the container and a respective sampling head and forms at least a portion of the filter chamber. The adapter fitting may be included with each container, or a group of adapters may be respectively associated with the corresponding group of sampling heads.

It is also important to provide structures and means for rotating an agitator in relation to the container and/or the sample in the container. As described in more detail below, an exemplary device according to the present invention may include a cover within a cover, wherein the agitator is fixed to a stationary inner cover and the container and outer cover freely rotate. Such relative motion moves the agitator in relation to the sample, and disperses particulate matter in the fluid.

A device according to the present invention may also include structures that are configured for and/or are adapted to mix the specimen collected in the collection chamber. Exemplary structures include but are not limited to a collection chamber having a rotatable cover, or a portion of the cover that rotates; a cover or cover portion that is moveable in relation to the collection container; and a tube or the like that extends into the collection container, said tube including one or more elements that mix the specimen. The cover may also include a portion that fittingly engages a portion of the cover in a liquid tight seal. The cover may also include a portion that fittingly engages a portion of the cover in a liquid-tight but not fluid-tight seal.

In a preferred embodiment of the invention, a specimen cup includes a chamber for collecting a liquid specimen, and in fluid communication with the chamber, a particulate matter separation chamber or module for separating particulate matter in the fluid and collecting the separated particulate matter in a collection zone. In a most preferred embodiment of the invention, the separated particulate matter is collected in a monolayer on the collection zone. A preferred embodiment of the invention also includes a hollow tube in fluid communication with the particulate matter separation chamber. More preferably, the hollow tube includes means for mixing the specimen and/or dispersing the particulate matter in the specimen. Exemplary means include but are not limited to an agitator, fins, brush, swab, broom, spatula, or the like. A preferred embodiment of the invention includes a tube having a brush. An exemplary brush is disclosed in U.S. Patent 4,759,376.

In accordance with the present invention, hollow tube 25 includes at least one agitator projection or fin 28 or the like, as shown in Figure 3. In one embodiment of the invention, hollow tube 25 is rotatable and agitator 28 stirs the liquid specimen, and in a most preferred embodiment, disperses cells and/or particulate matter, and/or disrupts any large particulate matter such as mucoid bodies. Agitator 28 may also include a body that is independent of the tube 25, and that is induced by a magnetic or electric field to stir the sample.

In an alternative embodiment of the invention, agitator 28 may comprise fibers, a brush, swab, or broom or the like. Preferably, such fibers or brush is are suitable for dispersing particulate matter in the container when the sample is vortexed in relation to the agitator, brush, or broom. In a most preferred embodiment of the invention, the brush or broom is also suitable for use in collecting particulate matter from a patient, e.g., a cervical brush or broom or the like. It is intended that the brush can be fixed to a portion of the cover, or the cover may include a slot or the like for matingly engaging a portion of the handle on the other end of the brush.

In one embodiment of the invention, a slot or opening in the cover can be covered with a removable or penetratable covering that protects the inside of the container from contamination until the container is ready for use. For example, a brush or the like can be used to collect a cervical sample, the covering can be removed from the cover, and the brush can be placed in the container.

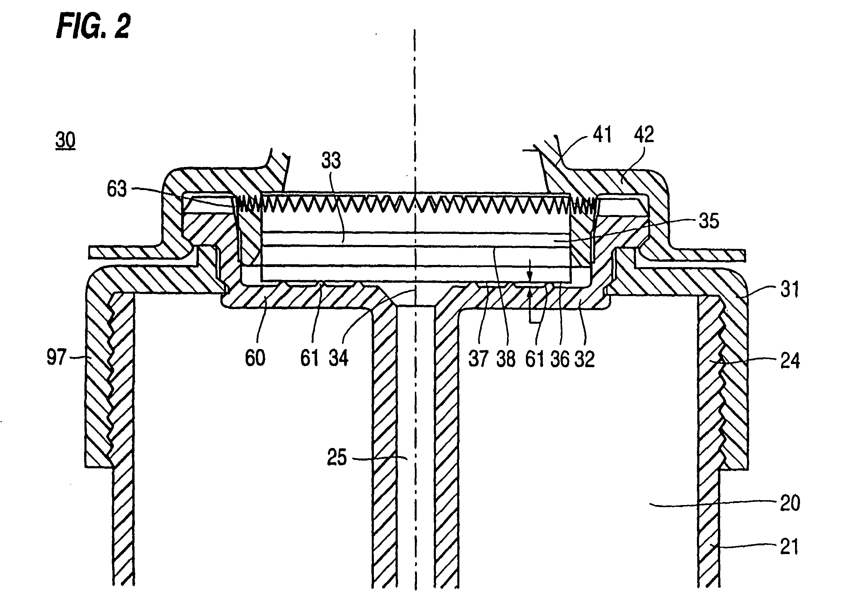

As noted above, an upper portion of the container and a lower portion of each head assembly matingly form a filter chamber. The containers and heads may be variously configured. Exemplary configurations are shown in Figures 2-5. In certain embodiments of the invention, the chamber 30 includes a base portion 31 formed in part from or engaged with the cover of the specimen container 20.

Base portion 31 also defines a well 32 suitable for seating a filter assembly 33. Well 32 is provided with a channel 34 or the like communicating with hollow tube 25. Well 32 may be an integral structure of base 31, or may be a separate structure. In certain embodiments of the invention, well 32 is a separate structure that is capable of rotating in the base 31. In order to achieve ease of relative rotation while maintaining a fluid-tight assembly, well 32 may matingly engage base 31 through a tongue and groove arrangement (see Figure 2).

As shown in Figures 2 and 3, the filter chamber 30 is preferably a two-piece housing formed by a top portion 41 at a lower end of a head, and a base portion 31 at a portion of the specimen container. In embodiments of the invention, top portion 41 releasably engages base portion 31; any housing configuration or assembly providing access to the porous arrangement 35 is suitable. Top portion 41 and base portion 31 may be connected or fastened to each other by any mating connection or means that provides a fluid-tight fit, e.g., Luer-type (threaded or not threaded), screw thread-type, friction-type, a tapered mating connection, or snap fit (as illustrated).

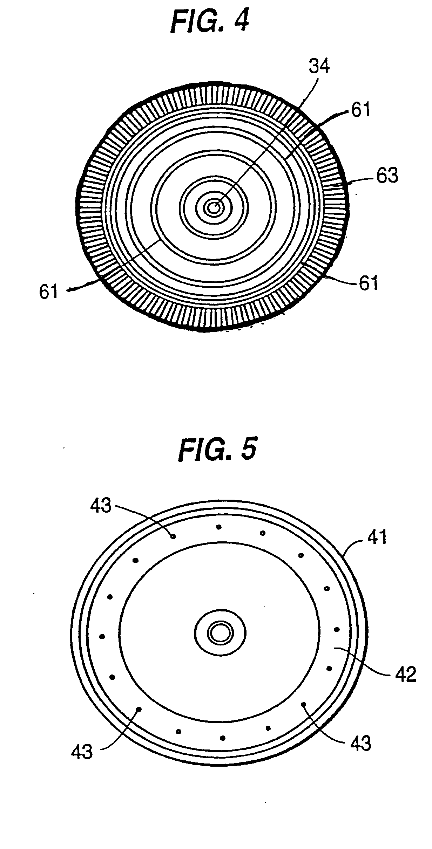

In accordance with embodiments of the invention, the well 32 of base 31 includes a seat 60 with one more spaced projections 61 or the like. Projections 61 are preferably of a size and shape sufficient to prevent porous arrangement 35 from flushly contacting seat 47. In the illustrated embodiment, projections 61 are concentric rings (see Figure 4). As will be described in more detail below, projections 61 break the surface tension between porous arrangement 35 and seat 60 so that, during use, when porous arrangement 35 is pulled away from seat 60, first porous medium 36 does not remain in contact with seat 60.

In one embodiment of the invention, projections 60 function in one or more of the following ways: projections 60 may break the surface tension between porous arrangement 35 and seat 39 so that, during use, when porous arrangement 35 is pulled away from seat 39, first porous medium 36 does not remain in contact with seat 39; projections 60 may evenly distribute pressure of the porous arrangement in the housing; projections 60 may prevent or suppress compression of the porous arrangement; and projections 60 may be configured to distribute any collected particulate matter in a pre-determined configuration or spatial distribution.

In accordance with the present invention, the surface of seat 39 may include one or more structures, configurations, or surface textures that promote the ability of the porous arrangement to release from the seat, that promote a pre-determined spatial distribution of particulate matter on the collection site, and/or prevent or suppress compression of the porous arrangement. One embodiment of the invention includes concentric projections, such as projections 60 described above. In another embodiment of the invention, the surface of the seat is configured into a sun-dial or clock face structure. Both of these embodiments, as well as other surface configurations disclosed herein, promote the collection of particulate matter on the collection site in a pre-determined spatial arrangement. Other configurations include, but are not limited to a grid, cross-hatching or the like, concentric squares or rectangles, or a series of continuous or separated structures, nubs, protuberances, granulations, or the like. It is intended that any element, structure, or chemistry that provides a texture to the surface of the seat is suitable for use with the present invention.

In accordance with another embodiment of the invention, the seat 39 and/or lower portion 32 may optionally include a channel or the like. In one embodiment of the invention, seat 39 slopes slightly outward toward the channel. The slight slope of the seat and the channel promote enhanced fluid flow through the housing and decreases the surface tension of the seat, both of which promote the capability of the porous arrangement to disengage from the lower portion 32 of the porous arrangement housing. This aspect of the invention is another structure(s) that promote release of the porous arrangement.

In one embodiment of the invention illustrated in Figure 5, the filter chamber assembly includes a top portion 41 that engages base 31, and in combination, forms filter chamber 30. Portion 41 includes a seat 42 or the like configured to engage porous arrangement 35. In embodiments of the invention, seat 42 positions porous arrangement 35 in well 32 so that porous arrangement 35 does not move while the sample is being drawn from its respective container. In certain embodiments of the invention, seat 42 includes a plurality of projections or posts 43 of a size, shape, and number so as to position the filter assembly in the filter chamber 30, to promote substantially even distribution of pressure against the porous arrangement, and to reduce or prevent compression of the porous arrangement that would interfere with fluid flow through the porous arrangement. Alternatively or additionally, porous arrangement 35 may include a serrated portion 63, as shown in Figures 2 and 3 that reduces or prevents compression of the porous arrangement.

### Filter Assembly

In accordance with the invention, the filter assembly chamber 30 is configured to receive a porous arrangement 35 having a particulate matter collection site 36 adapted to collect particulate matter as the sample containing the particulate matter passes through the chamber 30.

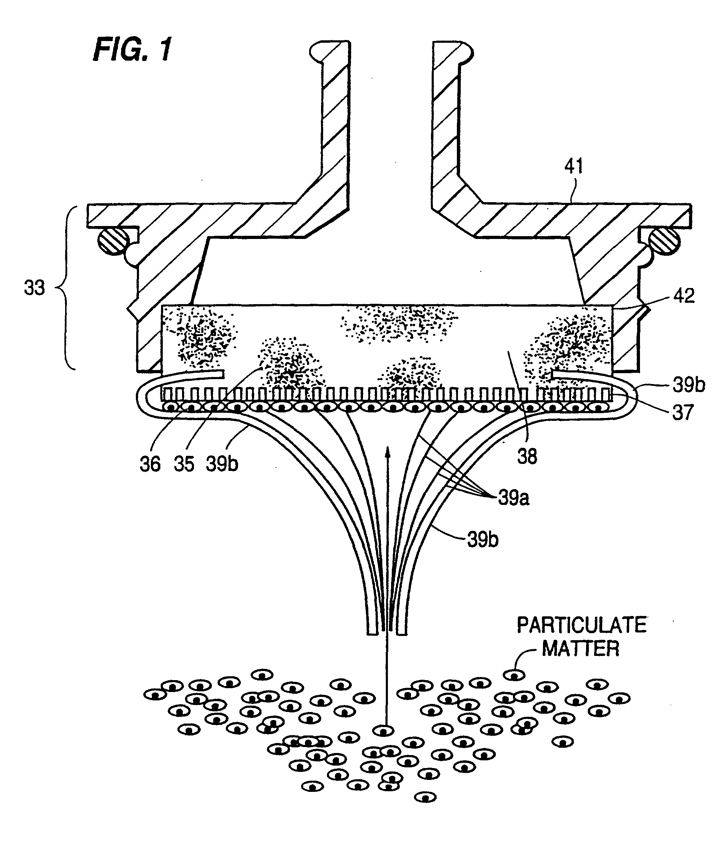

Porous arrangement 35 having a collection site 36 adapted to collect matter may be positioned in the fluid flow path such that the collection site 36 communicates with hollow tube 25. The porous arrangement 35 within the filter chamber is preferably adapted to define first and second branches of the fluid flow path. The first branch 39a extends through the collection site 36 and the second branch 39b bypasses the collection site 36 (see Figure 1).

In embodiments of the invention, the porous arrangement 35 includes a first porous medium 37, suitable for preventing the passage of matter therethrough, and a second porous medium 38, suitable for allowing the sample to pass therethrough. The second porous medium may or may not be capable of removing particulate matter from the sample, according to the needs of a particular device. The first porous medium is suitable for capturing or collecting solid matter in a uniform, single layer, i.e. a monolayer. The second porous medium is suitable as a support for the first porous medium.

Preferred porous media are disclosed in U.S. Patent 5,301,685 and U.S. Patent 5,471,994.

The nature of the material used to make the porous media, the compatibility of the materials chosen for the porous media with one another and with the liquid to be processed are all factors to be considered in selecting a particular material for a porous medium for a given application.

The first porous medium and the second porous medium may be positioned in any fashion that functions as described herein. As one skilled in the art will recognize, the porous arrangement may be variously configured and positioned as needed to achieve a particular result. For example, the first and second porous media may be separate, spaced apart media; the two media can be laminated together; the first medium can be integral with or removably engaged with the second porous medium; or the collection element may comprise a zone of higher density which mimics the function of the first porous medium as described above, and a zone of lower density which mimics the function of the second porous medium as described above. Choice of these various configurations is well within the skill of practitioners in the art. Variations on the structure and composition of the porous arrangement will be described in more detail below.

Porous arrangement 35 may include a unitary structure having a first portion of density and/or pore size suitable to prevent the passage of cells therethrough and a second portion of density and/or pore size suitable for passing the fluid therethrough.

In some embodiments, the porous arrangement includes a first porous medium comprising a porous polycarbonate membrane, suitable for preventing the passage of cells therethrough, and a second porous medium comprising a depth filter, or frit. The frit may be made of polypropylene or high-density polyethylene POREX7 porous plastics.

It should be noted that various types of porous arrangements can be used interchangeably with that of the present embodiment. While a polycarbonate membrane is especially suitable for use in the cytology collection apparatus of the present invention, other porous membranes are also suitable.

The porous membrane preferably has a pore size from about 0.22 microns to about 8 microns, more preferably from about 1 micron to about 6 microns, most preferably about 2 microns, which allows it to trap cells which are more than 3 microns in size. The membrane is suitable to allow fluid flow to pass therethrough while preventing the passage of particulate matter. The second porous medium is suitable for passing fluid therethrough and may also be capable of removing particulate matter from the sample. The pore size of the second porous medium may range from about 5 microns to about 60 microns, preferably from about 15 microns to about 45 microns, most preferably about 35 microns.

As one skilled in the art will recognize, adjusting the pore size of the porous membrane and the porous depth filter in accordance with the type and/or size of matter to be collected permits the collection of the matter on the collection site 14. The pore size is chosen so that a a monolayer of matter is formed on the collection site. For example, from about 3 µm to about 40 µm or more has been shown to be effective, but it is intended that the invention should not be limited to a certain range of pore size.

In embodiments of the invention, first porous medium 37 may be attached to second porous medium 38 using an adhesive that is soluble in the sample. Such soluble adhesives include but are not limited to sugar compositions, gels, and the like.

While the cytology collection apparatus 10 can be used for any biological fluid, it is particularly useful for preparing test samples from urine and its associated cells, and for Pap smears.

### Sample Container Support

A group of the specimen containers is positioned by hand or mechanically on a container support for further processing in an automated apparatus according to the present invention. The container support is preferably a substantially planar platform, disc, sheet, shelf, tray, or the like. In embodiments of the invention, a sample container includes guides suitable for positioning and/or retaining one or more specimen containers. In certain embodiments of the invention, the container support includes one or more recesses or cavities adapted to accommodate at least one size of sample container. In a preferred embodiment of the invention, the container support includes at least two recesses for accommodating a least two different size sample containers. The container support is preferably movable, i.e., adapted to rotate around an axis or be translated along a path. In accordance with the invention, the container support is movable to determined positions or stations, including one or more stations that align a portion or portions of the container support adjacent to or in proximity to another element of the automated apparatus, e.g., a sampling head, a loader or an unloader.

According to one embodiment of the present invention, the sample containers may be rotated within the container support, and a portion of the container cover fixedly associated with the agitator may be held relatively stationary with respect to the rotation of the containers.

### Stirring Assembly

A mixer according to embodiments of the invention includes an agitator for stirring each sample in its respective specimen container. As noted above, each head may include a portion that engages a stirring element extending into the sample. This portion of each head is adapted to be connected to a movement transmission, e.g. a motor rotating a belt, or the like that will rotate the portion of each head. The movement transmission may engage a single head, or preferably; engage all of the heads. The belt of a movement transmission may alternatively be activated by rotation of the specimen container supports around an axis.

According to one embodiment of the present invention, structures engage a portion of the cover of a collection container, and one or more structures rotate the collection container(s). The apparatus may be configured to process a single sample container or more than one, and may be operated semi-automatically, or automatically.

In some embodiments of the invention, the cover may include an outer stationary cover and an inner rotatable cover. In a certain embodiment of the invention, the inner and outer covers are not rotatable with respect to each other in a first position, and are freely rotatable with respect to each other in a second position.

According to one embodiment of the present invention, a structure or element rotates the sample container and another structure or element engages a portion of the sample container, e.g., a portion of the cover, for holding that portion relatively stationary.

Some embodiments of the invention include a cover having an inner portion or cover and an outer portion or cover, and wherein closing the cover and releasing one of the covers or portions so that it is freely rotatable occurs in a multi-step procedure. When the inner and outer covers are used to seal the container, it is preferred that the inner and outer covers do not freely rotate in relation to each other. Both covers seal the container up to a first position. In a second position, a catch or seal or the like is broken, e.g., by an additional twist of the outer cover, thus freeing the outer cover from its connection with the inner cover. In the second position, the outer cover rotates freely in relation to the inner cover.

In one embodiment of the invention, the cover comprises a first portion that fixedly engages the container and a second portion that may be rotatable in relation to the container. As used herein, rotatable in relation to the container refers to the relative movement of the first portion and the second portion; the first portion may be fixed and the second portion moveable, or the first portion may be moveable and the second portion fixed. In a certain embodiment, the second or inner portion of the cover is stationary and the first or outer portion is rotatable. In one embodiment of the invention, the agitator is engaged by or fixed to the second portion of the cover.

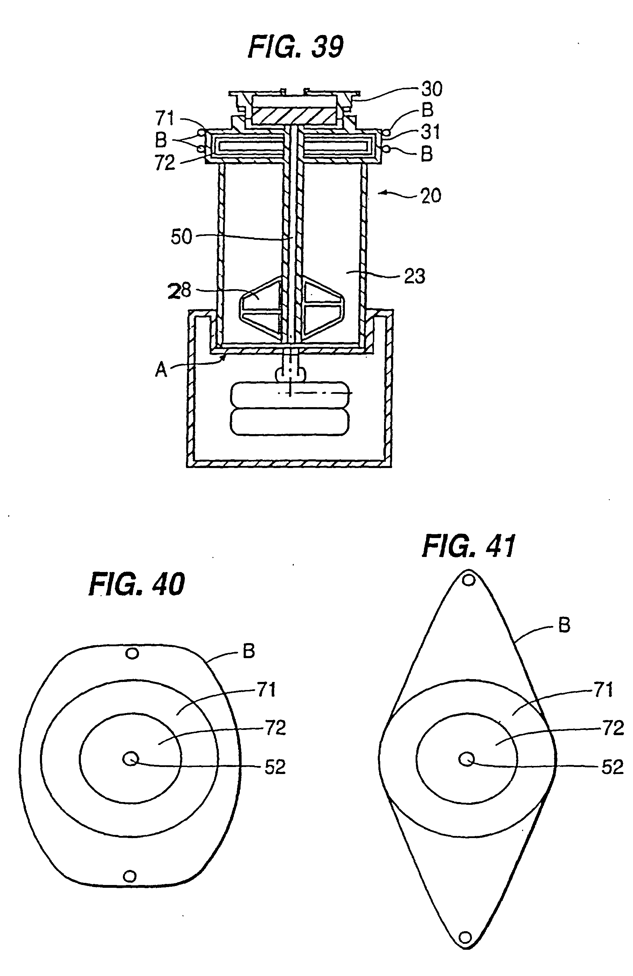

In accordance with the invention, first and second portions may be individual covers that matingly engage. For example, referring to Figure 39, inner cover 72 may be used to seal the container and outer cover 71 may snap fit over the inner cover. In this embodiment of the invention, a tab or the like on the inside of the outer cover may prevent relative movement of the inner and outer cover when the respective covers are in a first position.

Moving the outer cover to a second position, e.g., breaking the tab or by removing a spacer allowing axial relative axial displacement of the inner and outer covers, permits rotation of the outer cover relative to the inner cover.

An apparatus of the present invention may be configured to support, engage, and rotate a portion of a collection container so that the sample is mixed according to the present invention. An exemplary collection container includes a container or cup suitable for collecting and holding a specimen sample, a cover having a first position that is not rotatable in relation to the container and a second position that is rotatable in relation to the container, and an agitator engaged by or fixed to a portion of the cover and extending into the container.

As used herein, configured to support, engage, and rotate refers to various configurations that may be adapted to perform the specific function. For example, an apparatus according to the invention may include a container support for positioning at least one sample container and rotating the chamber portion of the container, and a sleeve or clamp for engaging and fixing a portion of the cover that communicates with a dispersing element. Alternatively, the support may hold the container in a fixed position and a pulley, sleeve, or clamp may engage and rotate the portion of the cover that engages the agitator. In one embodiment of the invention, the sleeve engages an inner or second portion of the cover, and holds the second portion of the cover in a stationary position in relation to the first portion of the cover.

As shown in Figure 39, cover 31 includes structures and means for allowing an outer portion of the cover to move in relation to an inner portion. As illustrated, cover 31 includes outer cover 71 and inner cover 72. Inner cover 72 is preferably fixed to or in fluid communication with tube 50. In one embodiment of the invention, cover 71, when tightened to container 23 rotates about inner cover 72 and tube 50. Such relative motion between outer cover 71 and inner cover 72 moves sample in the container 23 in relation to agitator 51.

In one embodiment of the invention, a slot or opening in the cover can be covered with a removable or penetrable covering that protects the inside of the container from contamination until the container is ready for use. For example, a brush or the like can be used to collect a cervical sample, the covering can be removed from the cover, and the brush can be placed in the container. An exemplary brush is disclosed in U.S. Patent 4,759,376.

### Sampling Station

An apparatus according to the invention also includes a group of sampling head assemblies adapted to engage a portion of corresponding specimen containers. According to the invention, the number and arrangement or pattern of the groups of heads and specimen containers correspond with one another. In embodiments of the invention, each head assembly includes a portion having a cavity receiving or engaging a filter as described below. In certain embodiments, a portion of the sampling head assembly matingly and removably engages a portion of the cover, and in mating engagement, forms a chamber adapted to position and accommodate a filter assembly. In accordance with the invention, the filter assembly and chamber provide at least two fluid flow branches through the chamber. A portion of the sampling head assembly is preferably movable in a direction to engage the specimen containers on the container support. In accordance with certain embodiments of the invention, container supports are sequentially moved with respect to a stationary reference frame into a position to be engaged by the sampling heads.

In other preferred embodiments of the invention, an adapter fitting is interposed between the container and the respective head and forms at least a portion of the filter chamber. The adapter fitting may be included with each container, or a group of adapters may be respectively associated with the corresponding group of heads.

Each head assembly is connected to a pump or the like. In this embodiment of the invention, the various structures provide a fluid flow path from the specimen container, through the filter in the filter chamber, and away from the specimen container toward the pump.

### Slide Handling

An apparatus according to the invention also includes one or more slide supports. A group of the slides is then positioned by hand or mechanically on a slide support for further processing in the automated apparatus according to the invention. In embodiments of the invention, a slide container includes guides suitable for positioning and/or retaining one or more slides. The slide support is preferably movable, i.e., adapted to rotate around an axis or be translated along a path. In accordance with the invention, the slide support is movable to determined positions or stages, including one or more stages that align a portion or portions of the support adjacent to or in proximity to another element of the automated apparatus, e.g., a loader or unloader.

### Fixative

A composition according to the invention includes one or more solvents, preferably an alkanol, between about 35% and about 45% by volume; ketone, between about 2% and about 3% by volume; a diluent, preferably a diol or triol, from about 1% to about 3% by volume; a crosslinker, preferably an aldehyde, from about 0.4% to about 3% by volume; glycerol, from about 0.5% to about 2% by volume; one or more detergents and/or dispersing agents, preferably non-ionic, from about 0.01 % to about 0.05% by volume; and a buffer, from about 45 to about 65% by volume. In one embodiment of the invention, the pH of the composition is between about 4 and about 7.

One embodiment of the present invention also includes a method of preparing particulate matter, such as cells and the like, for cytological or histological examination comprising collecting particulate matter in a uniform layer, preferably a monolayer, and fixing the cells in a composition according to the present invention.

Table 1 summarizes the range and preferred concentrations of the components of a fixative formulation according to the present invention.

**TABLE 1**

| Component | range (by volume, %) | preferred (by volume, %) | example |
|---|---|---|---|
| solvent | 35 -- 45 | 37 -- 42 | alkanol |
| ketone | 2 -- 3 | 2.1 -- 2.4 | acetone |
| diluent | 1 -- 3 | 1.6 -- 1.9 | diol, triol |
| glycerol | 0.5 -- 2 | 0.8 -- 1.2 | glycerol |
| crosslinker | 0.4 - 3 | 0.6 - 0.8 | aldehyde |
| detergent | 0.01 - 0.05 | 0.02 | Nonidet P40 |
| buffer | 45 - 65 | 50 - 55 | Tris |

A composition according to the invention includes one or more solvents to penetrate the tissue or cells, dehydrate the cells, and/or inhibit bacterial and vital activity. In one embodiment of the invention, the solvent is a mixture of alkanols, which penetrate slowly, and when combined with other reagents, fixes the sample rapidly. It denatures protein by precipitation, precipitates glycogen, and dissolves fats and lipids. The alkanol can be any of thee well known alcohols having one to four carbons, e.g., methanol, ethanol, n-propanol, isopropanol, n-butanol, and various branched butanols. The most preferred solvent is a mixture of methanol and isopropanol, typically about 30% and about 10% by volume, respectively.

The ketone is a fixative with similar action to that of alcohol, except that glycogen is not well preserved. The ketone acts as a fixative and additionally enables the overall composition to penetrate the cells. The preferred ketone is acetone.

The diluent forms a coating over the specimen and helps protect from the effects of drying. The preferred diluent is a diol or a triol, most preferably polyethylene glycol (PEG), e.g., PEG-1500 (average molecular weight of about 1450) also called Carbowax.

Glycerol prevents the drying of cells during sample processing. Cells that have been kept in fixative solution for an extended time typically become rigid due to the fixing process and are less able to spread on the slide. Glycerol helps the cells to flatten on the slide.

The crosslinker reacts with protein end-groups to crosslink molecules and produces an insoluble product. Protein groups involved include amino, imino, and amido, peptide, hydroxyl, carboxyl and sulfhydryl. Methylene bridges are also commonly formed between similar groups such as NH2 and NH but are thought to be reversible by washing in water. Some crosslinkers such as formaldehyde are an antiseptic.

The preferred crosslinkers are aldehydes, most preferably formaldehyde.

The detergent is non-ionic detergents and dispersing agents used for solubilization of proteins and membrane components to diminish cellular aggregation. The preferred detergents are Nonidet P40 or Triton X-100, both well-known detergents.

The buffer maintains the solution at a pH between about 4 and about 7, and provides a medium for transportation. The preferred buffer is Tris, a well-known buffer. In accordance with the present invention, the buffer may also include a fixative that precipitates nucleoproteins and one or more osmolarity maintainers. The preferred nucleoprotein precipitator is acetic acid glacial, typically in a range from about 0.2% to about 0.3% by weight, and helps maintain the buffer between about 7.4 and about 7.8 pH. The preferred osmolarity maintainers are dextrose, typically in a range from about 0.1 % to about 0.2% by weight, and sodium chloride, typically in a range from about 0.7% to about 0.8% by weight.

In one embodiment, the active fixative ingredients described may be dissolved in a suitable solvent such as distilled water, and this solution can then be used as a fixative agent in a number of ways as would be obvious to one skilled in the art. For example, the fixative solution can be used to preserve samples of tissue that are being shipped or carried to an examination site. In this process, small vials or jars that have liquid right seals are filled with the reagent of the invention, and tissue samples are placed in the reagent-containing vial to preserve the samples until they reach an area where further processing can occur. Water or other diluent is also used in an amount of about 80 to about 90 percent by volume. Any suitable diluent that does not change the important chemical and physical characteristics of the formulation may be used.

Tissues prepared for study using the fixative of the invention can be prepared for histological study in any known conventional manner, such as through the use of paraffin, sectioning equipment, staining, mounting on slides, or other common steps utilized prior to microscopic or other examination. The present invention thus provides a safe, convenient and effective fixative solution that can be utilized in the many known histological procedures that employ such solutions.

### Fluid Movement Structures

In accordance with embodiments of the invention, the automated apparatus includes one or more elements for altering differential pressure within the apparatus so that the sample can move through a portion of the automated apparatus. In accordance with embodiments of the invention, the fluid component of the sample may be either gaseous or liquid, depending on the use. For example, inducing a vacuum in a conduit communicating with the sample container will draw the sample in the container through the filter assembly. It may also be desirable to induce a positive pressure to return any uncollected portions of the sample to the specimen container, or to move the filtered liquid to a disposal container or chamber. A reversible pump is one example of a preferred vacuum/pressure element.

Additionally, it may be desirable to clean or rinse a portion of a sub-assembly, e.g., a portion of the head assembly. In embodiments of the invention, the pump or the like may move a rinse solution from a source container through a conduit to the head assembly. Included within the present invention are a variety of source containers, pressure differential generators, and conduits for establishing fluid communication between or to pre-selected elements of the automated apparatus.

Movement of a fluid through the system may be effected by maintaining a pressure differential between a source of fluid and a destination of the fluid. Exemplary means of establishing this pressure differential may be by applying pressure to any part of the system on the inlet side of the filter chamber; applying a vacuum to any part of the system on the outlet side of the filter chamber; or any form of pump, such as an autovial spunglass filter (manufactured by Genex Corporation); gravity head; or a flexible, collapsible container, such as a specimen container, which may be squeezed to force the sample through the filter.

### Controllers

In accordance with embodiments of the invention, the automated apparatus may include one or more controllers for selectively moving and positioning an element of the automated apparatus, for initiating or discontinuing an operation of the automated apparatus, or for monitoring the progress of the operation of the automated apparatus. An example of a preferred controller is a computer and computer program. Other uses of a controller will be evident to one skilled in the art, and are included within the invention. Exemplary controllers are described in more detail below.

### Loaders

In accordance with embodiments of the invention, at least one loader may be adjacent to or a part of the automated apparatus. It is intended that a variety of loaders may be used in conjunction with the operation of the automated apparatus. For example, an automated apparatus may include one or more of the following: a filter loader; a microscope slide loader; a specimen container loader; a capper, designed to position and place a cover on an open specimen container; a loader for positioning and matingly engaging a portion of the head assembly on a group of the specimen containers; a loader for positioning and matingly engaging a portion of each filter assembly to respective slides. Exemplary loaders are described in more detail below.

### Unloaders

In accordance with embodiments of the invention, at least one unloader may be adjacent to or a part the automated apparatus. It is intended that a variety of unloaders may be used in conjunction with the operation of the automated apparatus. For example, an unloader may be used for any element for which there is a loader, as described above. Exemplary unloaders are described in more detail below.

### Tracking Mechanism

In accordance with embodiments of the invention, the automated apparatus may include one or more tracking mechanisms for tracking the progress of a sample through the automated apparatus, and/or to track a sample after it has been processed by the automated apparatus. An exemplary tracking mechanism involves the use of a bar code. In this embodiment of the invention, the automated apparatus may include one or more bar code readers and a printer. An exemplary tracking mechanism is described in more detail below.

### Miscellaneous Structures

An automated apparatus according to the invention may also include a variety of movement transmissions including belts, motors, pulleys, anti-friction elements, lifters, transports, and supports, as well as conduits, a rinsing or cleaning head and its source or supply (e.g., container) of a rinsing or cleaning solution, a fixative applicator and its source or supply (e.g., container) of fixative solution, and the like to effect operation of the elements of the automated apparatus. Other additional or substituted structures will be evident to one skilled in the art, and are included within the invention. Exemplary structures are described in more detail below.

### Method of Operation

It will be clear from the description of the various elements of the automated apparatus that a wide variety of methods of operation may be used. An exemplary mode of operation is described below, and it is intended that the nature, sequence, and number of steps are exemplary.

A group of specimen containers containing respective samples are arranged on a container support, either manually or by a loader. In some embodiments of the invention, a technician enters into the controller one or more of the following parameters for each specimen container: specimen type (e.g., sputum, blood, urine, spinal fluid, etc.) mixing rate, mixing time, suction level, suction time, fixative (e.g., either a yes or no value), and drying time. After the appropriate parameters are loaded into the automated apparatus, the apparatus initiates sample processing. In embodiments of the present invention, a bar code reader detects a bar code on each container, and the controller selects appropriate parameters based on bar coding on each container.

A filter assembly loader may be used to position an appropriate filter assembly (corresponding to information received from the bar code and the pre-selected parameters) into the respective filter chamber for each sample.

The container support is then advanced to a sampling stage, i.e. a predetermined position with respect to the head assembly, and the group of heads is then engaged with the corresponding group of containers.

Concurrently, a group of slides is arranged on the slide support in a pattern corresponding to the group of heads. The group of slides is advanced to a depositing stage wherein respective monolayers of particulate matter from each corresponding sample are transferred to the slide from its respective filter assembly.

The mixer is activated by the controller thus driving the agitators for stirring their respective samples. The controller then activates the pump to apply an appropriate vacuum in each head, thereby drawing at least a portion of each sample from its respective container.

After each sample has passed from the container through its respective filter assembly, the controller de-activates the pump, and the group of heads disengages from the corresponding group of containers.

The group of filters from their respective samples are transported to engage the corresponding group of slides, thereby transferring each monolayer of particulate matter to its respective slide, and then the filters are unloaded from their respective slides. In some embodiments of the invention, a portion of the filter assembly, typically the membrane. portion, remains engaged with the microscope slide; in other embodiments, the entire filter assembly disengages from the microscope slide.

In embodiments of the invention, a fixative applicator provides a supply of fixative to adhere the monolayer of particulate matter to its respective slide. The controller activates the fixative applicator, dispensing an appropriate amount (e.g., four drops) of fixative on the microscope slide. A blotter may be used to absorb excess fixative and to remove any portion of the filter that remains on the slide.

In embodiments of the invention, each microscope slide having a monolayer of particulate matter deposited thereon is marked, e.g. with a bar code, and the corresponding bar codes on the containers and the slides are indicated, such as by a printout from a printer. Thus, each monolayer of particulate matter can be associated with its respective sample using the coding on the container and the slide.

In embodiments of the invention, the slide support is advanced and the groups of slides are removed from the apparatus, either manually or with an unloader.

Embodiments of the invention may also include a blower or drier for drying the surface of each microscope slide and/or removing, i.e. blowing off, any residual portion of the filter such as the membrane (if present). Again, the controller may coordinate the operation of the blower/drier with the operations of the other mechanisms and sub-assemblies of the apparatus.

Embodiments may also include a rinsing cup that may then be aligned with each filter head. The controller activates the cleaning process so that any portion of each head requiring rinsing or cleaning is brought into contact with the cleaning solution.

Embodiments may also include a sub-assembly for recovering each container with its original cover or with an unused cover. Used covers may be moved to a disposal area. The covered specimen containers may then be removed, either manually or with an unloader, from the automated apparatus for storage. Again, the controller would coordinate all automated operations associated with re-covering and storing the containers.

### FIRST EXEMPLARY EMBODIMENT OF THE INVENTION

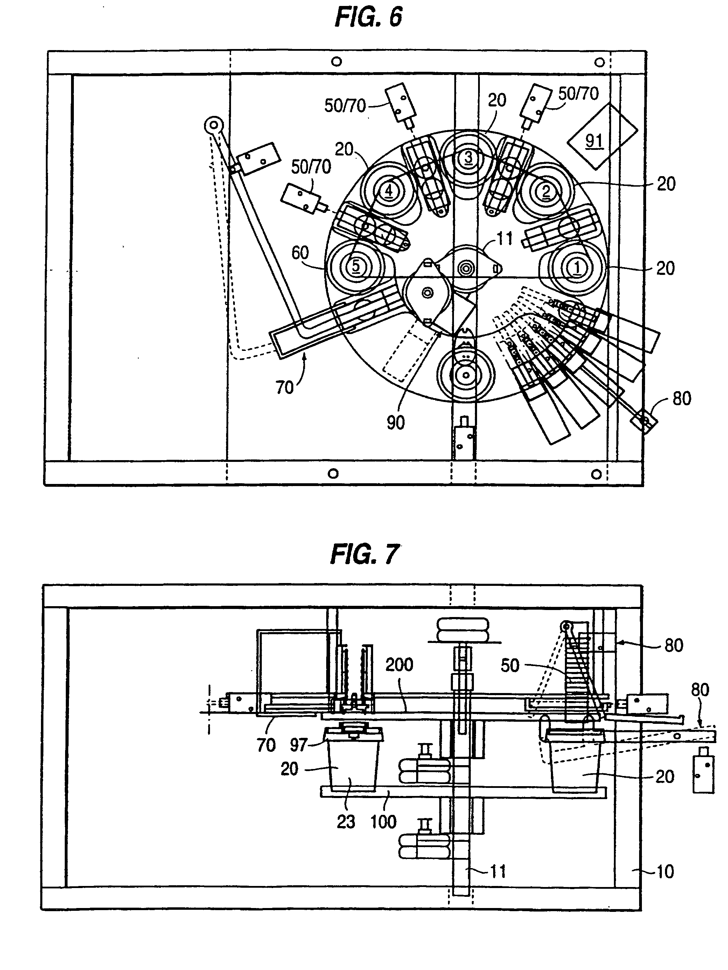

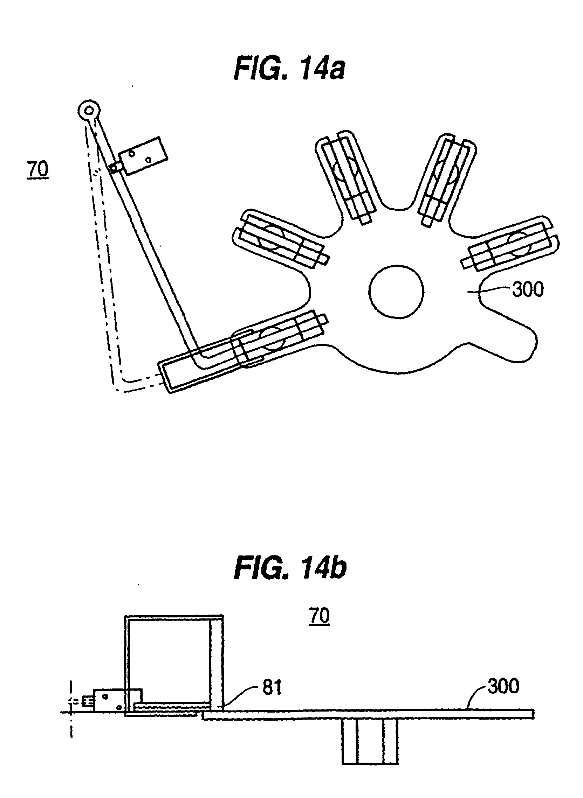

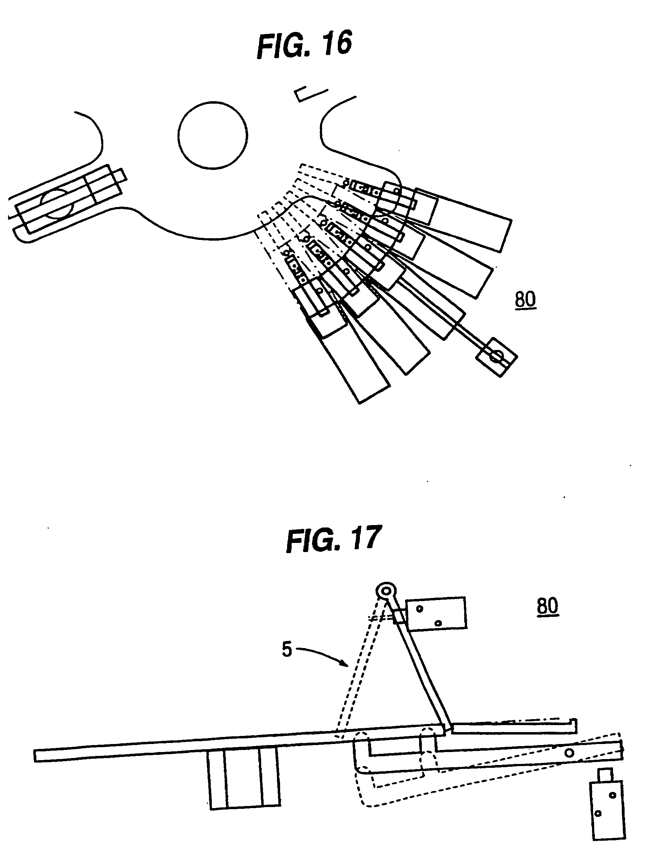

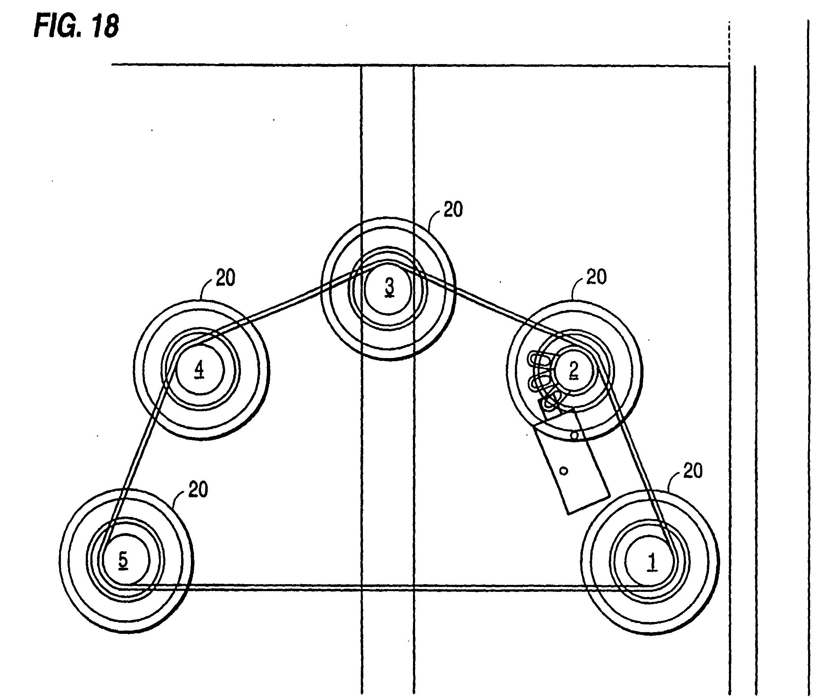

According to a first exemplary embodiment of the present invention shown in Figures 6-18, an automated apparatus 10 includes a group of specimen containers 20 arranged on a specimen container platform 100, a filter head assembly 40 positioned on a filter head support 200, and a microscope slide support 300. In the first exemplary embodiment of the invention, the specimen container support 100, the filter head support 200, and the microscope slide support 300 rotate around a central axis or shaft 11. As shown in Figures 6 and 7, the automated apparatus 10 may also include at least one filter assembly loader 50, microscope slide loader 70, microscope slide unloader 80, a bar code printer 90, a=bar code reader 91, a fixative container 92, an air container 93, a rinsing liquid container 94, and cleaning liquid container 95, a waste container 96, and a controller 400.

Each of these elements will now be described in more detail.

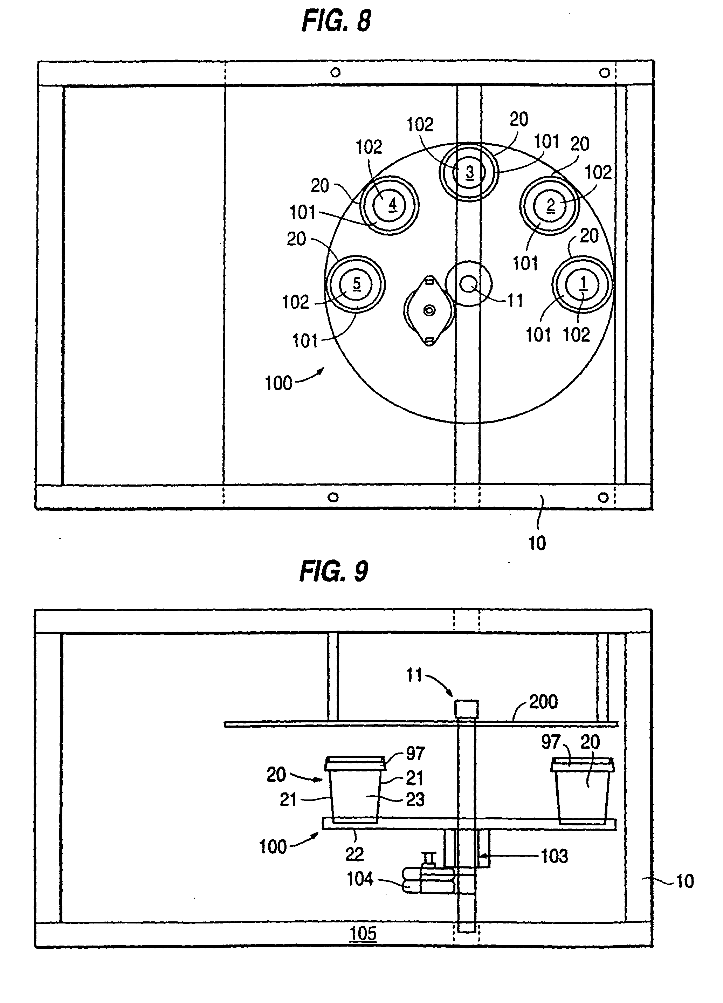

The specimen container support 100 as illustrated includes a group of five recesses 101 adapted to receive and position a specimen container 20. As shown in Figure 8, the recesses may be configured to receive more than one size specimen container, a large size 101 and smaller size 102. In the embodiment shown in Figures 8 and 9, the specimen container support 100 is circular and is movable up and down and axially around a central axis or shaft 11. To accommodate this movement, the automated apparatus may include one or more bearings 103 or the like, and a stepper 104 or similar element for moving the support 100 up and down the shaft 11. The specimen cup support 100 may move radially around the axis using a belt drive 105 or gear or the like.

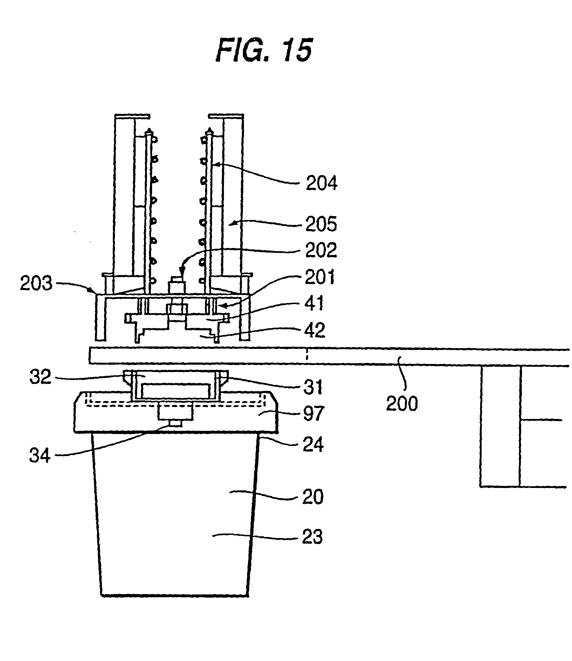

The head assembly 200 as illustrated in Figure 15 may include a lower portion 41 adapted to engage, position, and removably retain a filter assembly 33. In the first exemplary embodiment of the invention, the lower portion 41 is adapted to engage the filter assembly in a fluid tight or liquid tight seal, but such engagement is preferably releasable so that the filter assembly may be removed from the lower portion 41 during another step in the operation of the automated apparatus.

A portion of the head assembly 200 may include a gear or teeth 201 adapted to engage a belt or the like so that the lower portion 41 is rotatable. As noted above, this belt driven rotational movement is transferred to a portion of the specimen cup covers so that agitators extending into the container stir the sample.

The head assembly 200 also preferably includes a fitting 202 adapted to engage one or more conduits that establish fluid communication with at least one of an air container 93, a rinsing liquid container 94, a cleaning liquid container 95, and a waste container 96. The communication with the air container 93 may be used to disengage the filter assembly 33 from the portion 41 during another step in the operation of the automated device 10, or may be used to push a plunger or the like that pushes the filter assembly out of the portion 41.

As shown in Figure 15, the filter head assembly may also include one or more springs 203 for engaging a portion of the specimen container or cover 97, one or more springs 204 for allowing a portion 41 of the filter head assembly to move resiliently, and one or more bearings 205 that allow rotary movement of a portion of the head assembly. In most preferred embodiments of the invention, the head assembly 200 comprises a cylinder within a cylinder construction.

In embodiments of the invention, the slide support includes a plurality of radial projections adapted to receive a microscope slide with or without a filter. The slide support being rotatable about the same axis as the container support.

In accordance with embodiments of the invention, the slide support is positionable at an intermediate level between the container support and the head assembly. In accordance with the invention, the microscope slide support is movable to determined positions, including one or more positions that align a portion or portions of the support adjacent to or in proximity to respective filters.

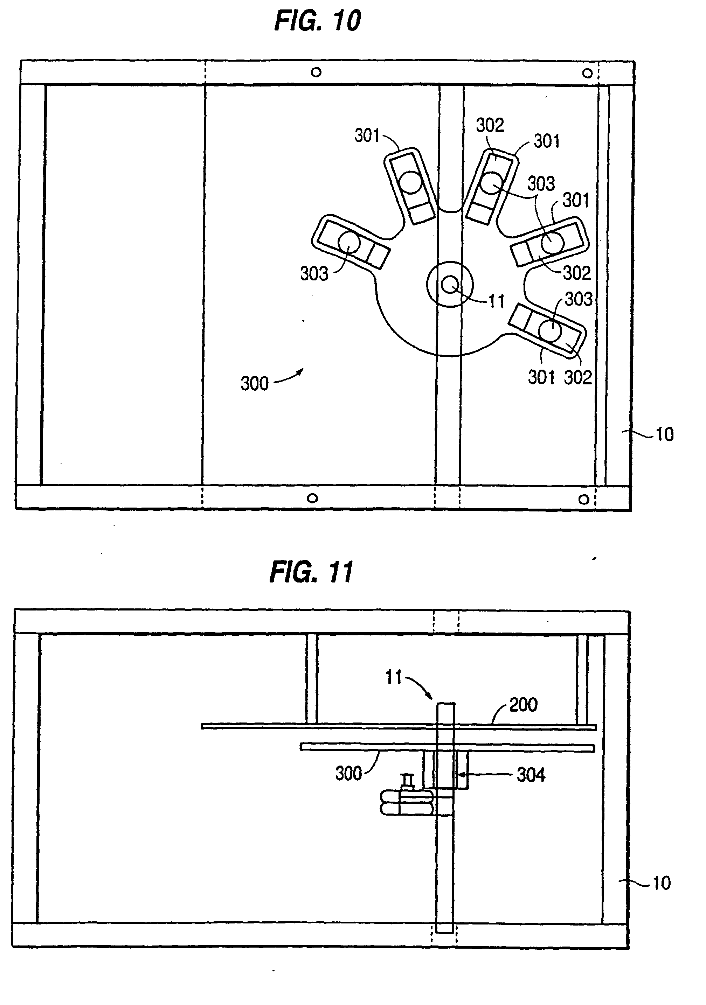

The microscope slide support 300, as shown in Figures 10-12 is adapted to receive a group of microscope slides and a group of filter assemblies 33. In a preferred embodiment of the invention, the support 300 includes radially extending projections 301. In a more preferred embodiment, each projection 301 includes a cavity or recess 302 adapted to receive and position a microscope slide, and each projection includes a cavity or recess 303 adapted to receive and position a filter assembly 33.

In accordance with the invention, the microscope slide support 300 is rotatable around the axis 11 and vertically along the axis 11. Bearings may be provided that facilitate movement of the support 300, and may also support cantilever loads or pressure on the projections 301 of support 300. The support 300 may also be rotatable by a transmission such as a belt drive, gears, or the like; and movable vertically using a stepper or the like.

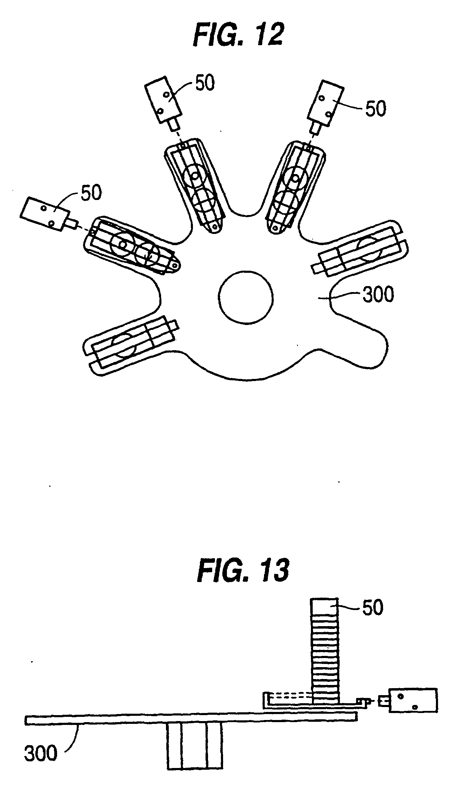

Filter loader assembly 50, as shown in Figures 7 and 13, preferably receives and positions groups of the filter assemblies 33, and is adapted to deposit one filter assembly 33 in a recess on the microscope slide support. In the first exemplary embodiment, filter assemblies 33 are stacked in a tube or channel, and a motor or solenoid moves a plate having a hole from a first position where the filter stack is closed to a second position where the filter stack is open. In the open position, a spring or the like may push a single filter assembly through the hole and onto the microscope slide support. Alternatively, the spring may be used to retain all but one of the filter assemblies, so that when the hole is aligned with the stack of filter assemblies, the un-retained filter assembly is allowed to drop onto the microscope slide support.

The microscope slide loader 70 preferably retains and positions groups of microscope slides, and is adapted to move a single microscope slide onto a recess in the microscope slide support. In some embodiments, the slides are arranged in a tube having a closed bottom end, and, adjacent to the bottom end, one or more slots 81 through which a single microscope slide can pass. An arm on the slide loader pushes a slide from the stack and onto the microscope slide support. When the arm retracts, the next microscope slide drops into position.

The present invention also includes a method for removing particulate matter from a sample, and for transferring a monolayer of the particulate matter, such as cells, to a microscope slide. This allows for clear observation and optimal diagnostic accuracy.

An exemplary method of using the invention includes collecting a sample containing particulate matter in a collection container 20. The container 20 is then capped with a cover assembly 500 that includes one or more of the following: base 31, well 501, and at least a portion of the filter chamber 30.

When the sample is pulled from the container 20, fluid will flow through porous arrangement 35 as shown in Figures 1 and 2, so that a monolayer of particulate matter is formed on collection site 36. Once the monolayer of cells is formed, fluid flow is reduced in the center of porous arrangement 35 and increases towards the edges of the porous arrangement. At least in part this is due to the blockage of flow by the collected cells as they form the monolayer on the surface of the porous arrangement. When the monolayer has mostly covered the surface 45 of the porous arrangement, the flow bypasses the first porous medium and passes through the extended side area of the second porous medium. Thus, the area of the second porous medium extending beyond an end wall or skirt of the top portion acts as a vent (with low resistance to flow) which prevents cells piling up or collecting in more than a monolayer. Fluid may be passed back and forth through the porous arrangement as many times as desirable.

The first porous medium may then be pressed against a microscope slide to transfer the monolayer of particulate matter on the collection site onto the slide. This allows a cytological examination to be performed on the cells by a practitioner without the interference of the pores in the membrane or delay due to processing requirements.

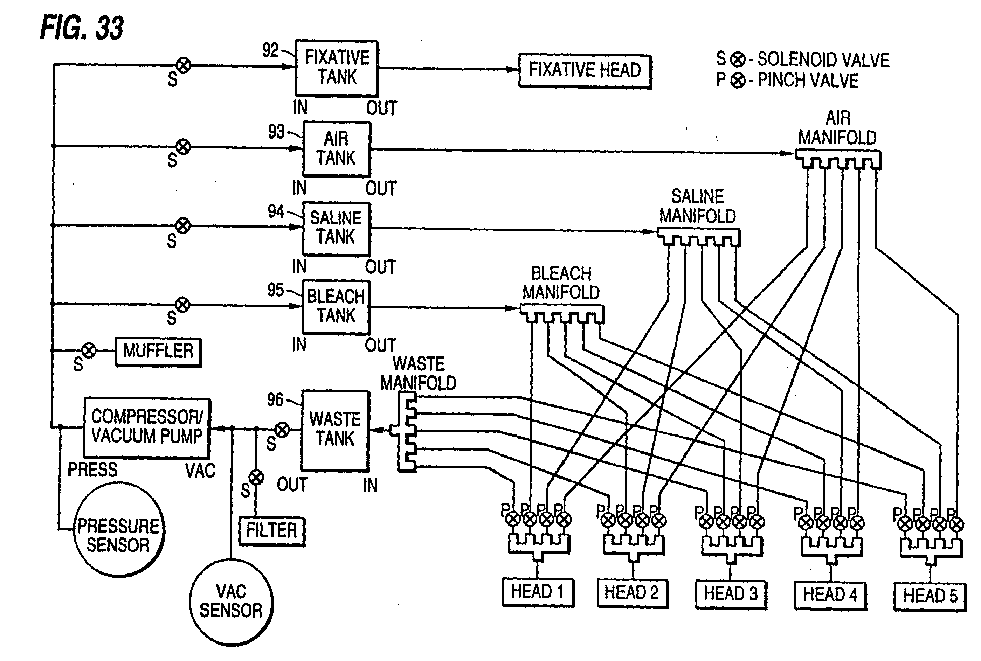

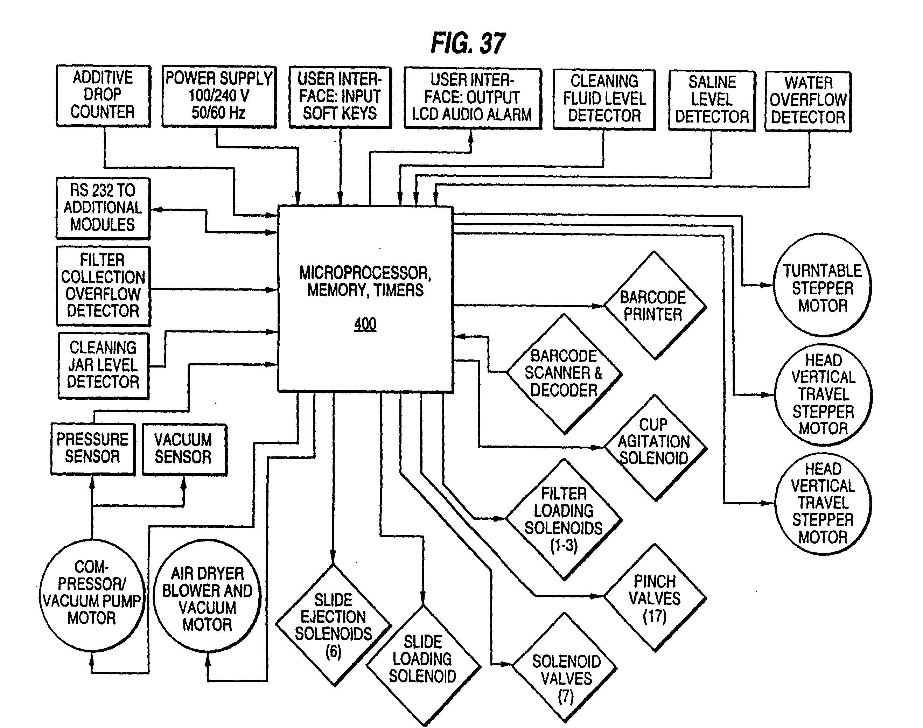

Figure 33 shows an exemplary fluid flow diagram according to the first preferred embodiment. Figure 37 shows an exemplary arrangement for controlling the process according to the first preferred embodiment.

### SECOND EXEMPLARY EMBODIMENT OF THE INVENTION

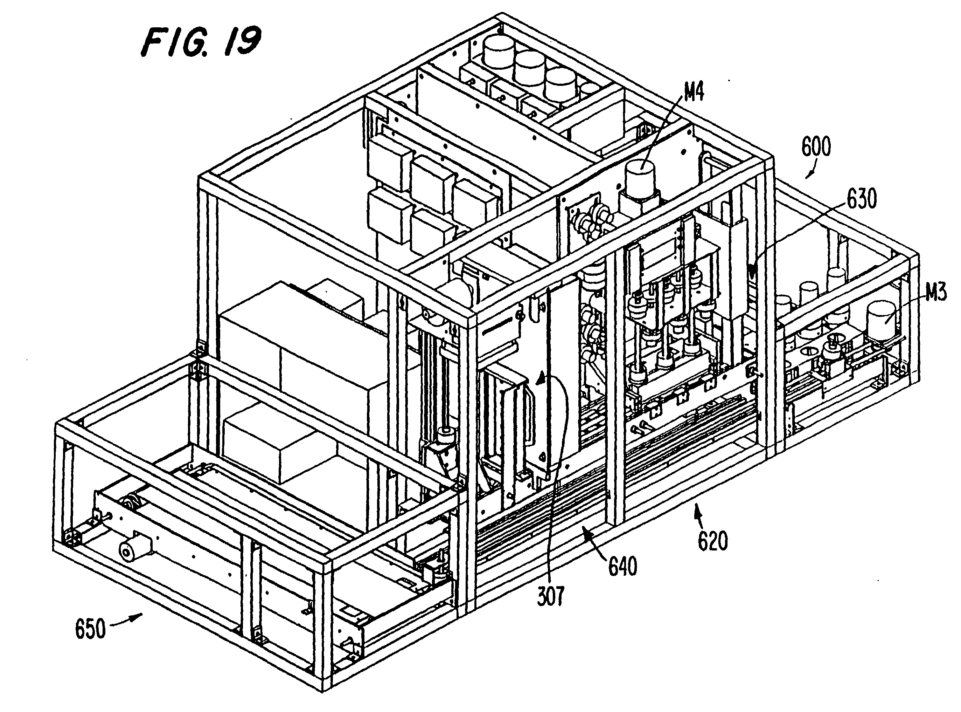

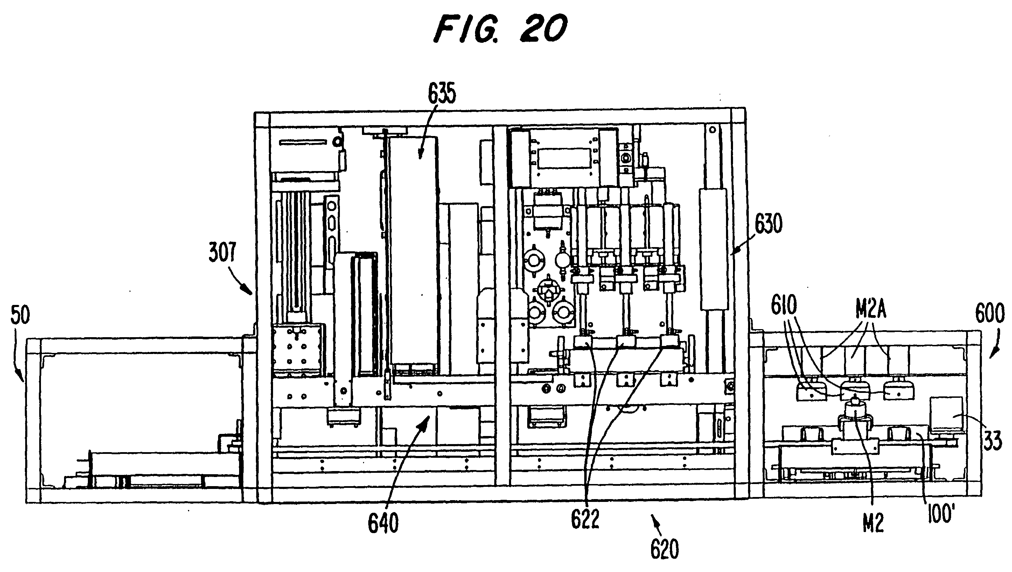

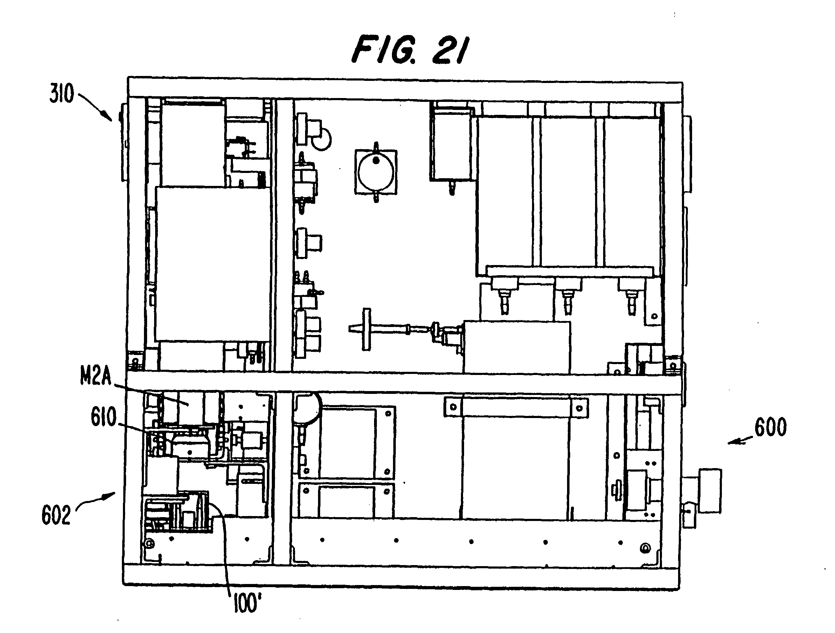

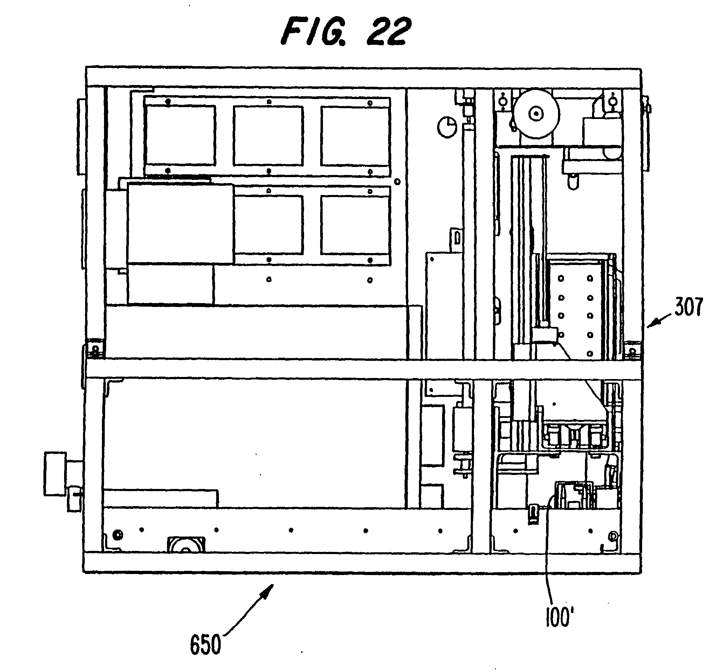

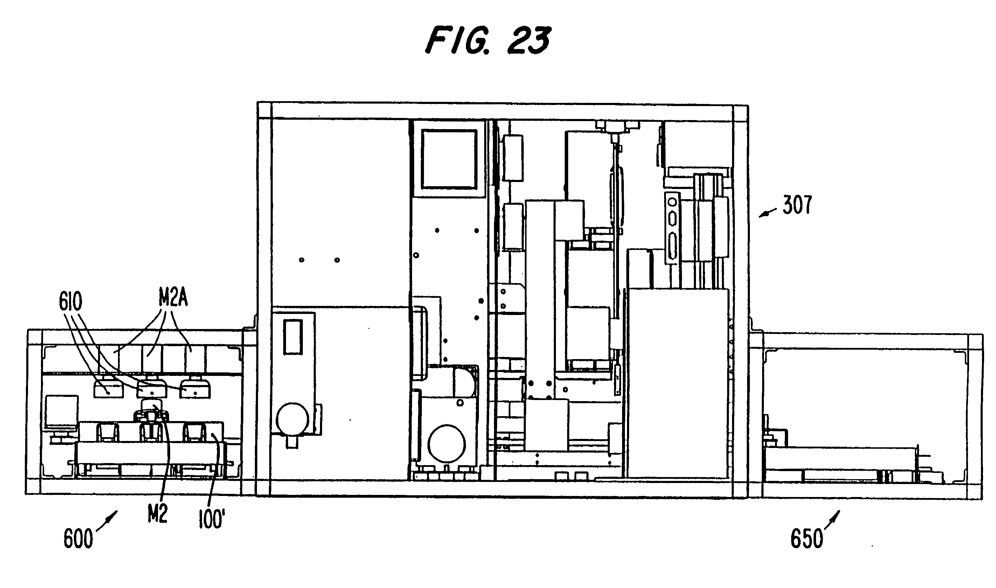

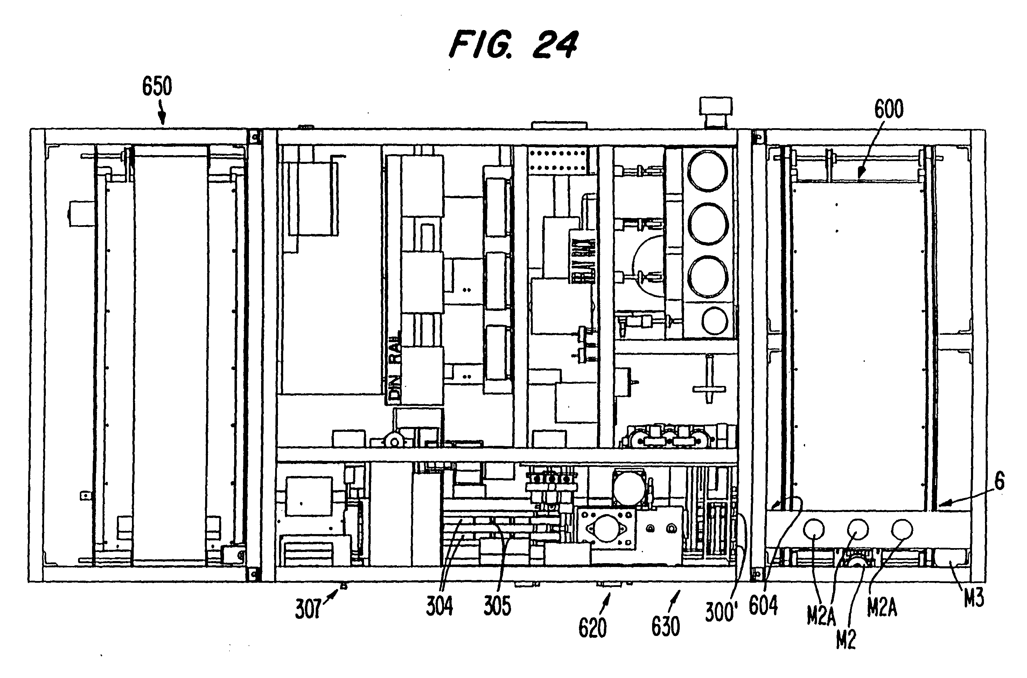

A second exemplary embodiment of the present invention shown in Figures 19-31C. A container support 100' arranges a group of three containers 20 in a linear pattern. The container support 100' cooperatively retains the containers 20 so as to prevent relative rotation between the containers 20 and the container support 100'. Alternatively, if the containers 20 are to be turned, the container support may facilitate rotation by providing access to the containers 20 (e.g., an aperture may be provided in the base through which a lifting pin is inserted so as to o provide a pivot axis for the containers 20).

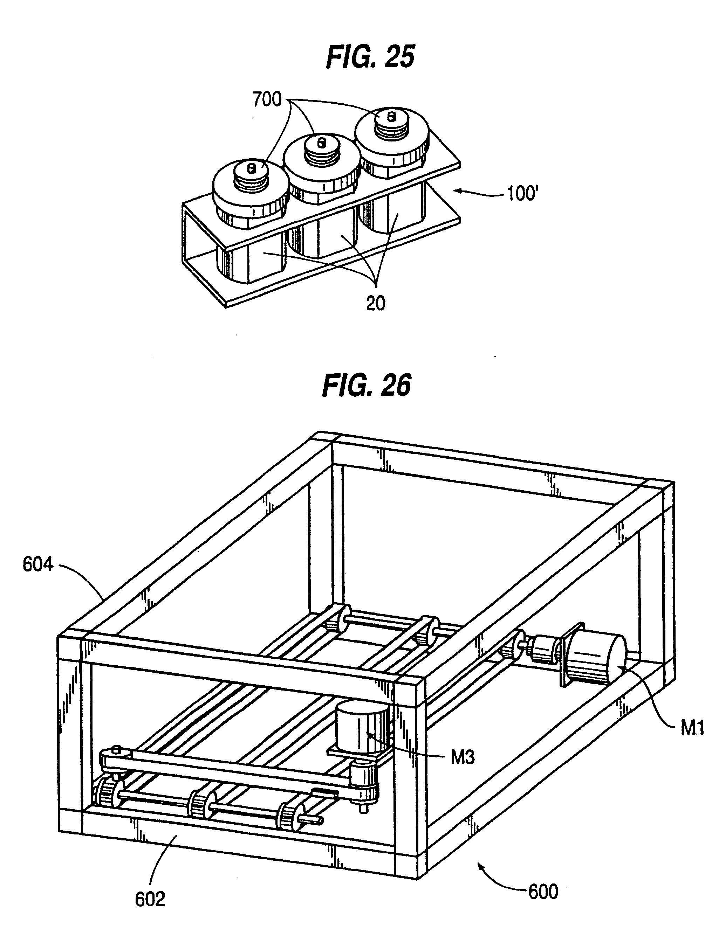

A container conveyor system 600 for advancing groups of containers 20 in their respective container supports 100'. A first transmission, including a motor M1 driving at least one belt (two are shown in Figures 19-24; three are shown in Figure 26), advances container supports 100' toward a front 602 of the conveyor system 600.

At the front 602 of the conveyor system 600, the samples in the containers 20 are agitated. According to a preferred embodiment, a transmission driven by a motor M2 raises the containers 20 into engagement with respective agitating motors M2A corresponding to each of the containers 20. Respective agitating heads 610 engages the covers of the containers 20. The containers 20 may be held relatively stationary, or may be allowed to rotate due to rotation of their covers by agitating motors M2A.

The degree of agitation may be selected according to the samples being tested. Motors M2A may be driven in accordance with various routines, and combinations thereof, including but not limited to: acceleration to a desired constant speed that is maintained for a predetermined amount of time before deceleration; variable acceleration and deceleration; and reversing the direction of rotation. After agitation is accomplished, the containers are lowered back into the container support 100' by the transmission driven by the motor M2.

Another transmission, including a motor M3 driving at least one belt, sequentially advances individual ones of the container supports 100' out a side 604 of the conveyor system 600 to a sampling station 620.

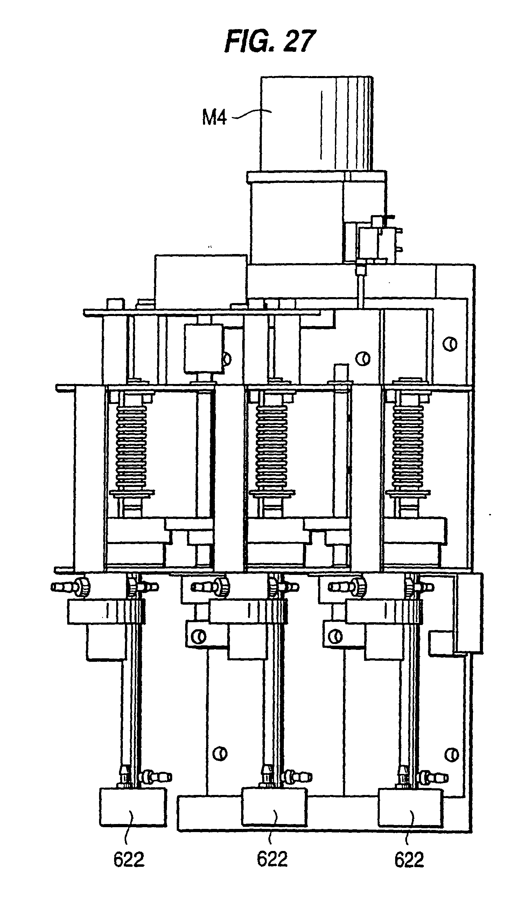

As best-seen in Figure 27, the sampling station 620 includes a set of sampling heads 622 (three are shown) that correspond to the number of containers 20 that can be processed in each batch. The arrangement of the sampling heads 622 also corresponds to the containers 20 in the container support 100', such that each of the containers 20 is aligned under a respective one of the sampling heads 622. The sampling heads 622 are vertically displaced, as a group, via a transmission (i.e., a threaded rod) driven by a motor M4. A spring system ensures adequate engagement of the heads 622 with respect to the containers 20.

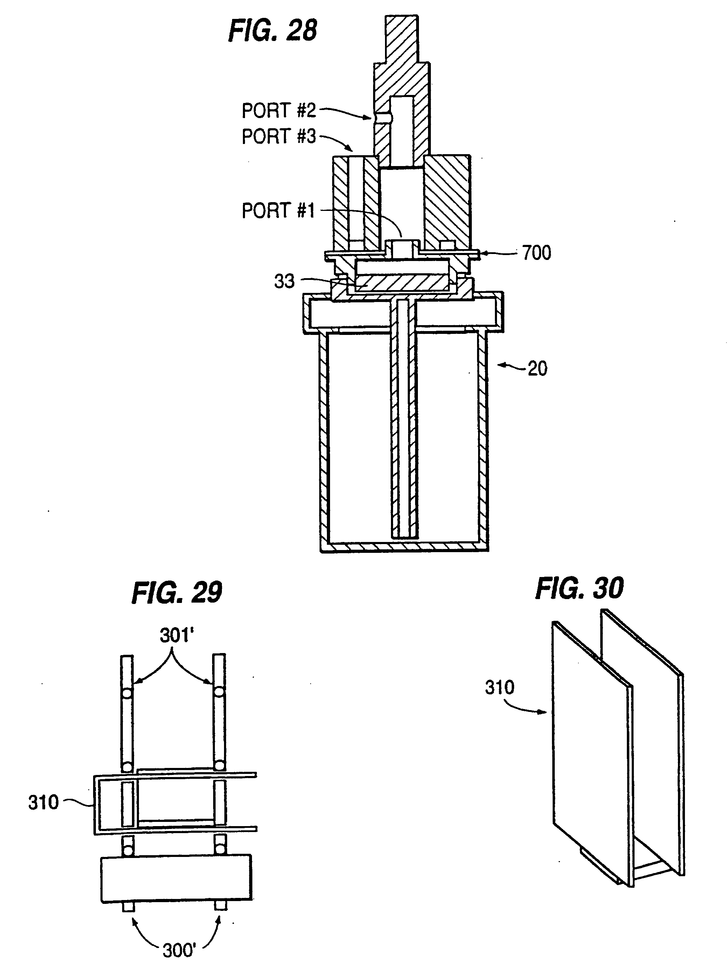

As shown in Figure 28, an adapter 700 matingly engages the container 20. The adapter 700 fixedly retains filter assembly 33, and is frictionally retained with respect to the container 20. Each sampling head 622 includes three ports: a first port for communicating with the sample via the adapter 700, a second port for communicating with the pump, and a third port for releasably engaging the adapter 700 to the sampling head 622. Connecting a vacuum source to the third port will cause the adapter 700 to adhere to the sampling head 622, enabling sampling head 622 to separate adapter 700 from container 20 against the frictional opposition retaining the adapter 700 with respect to the container 20. Disconnecting the vacuum source will allow gravity to separate the adapter 700 from the sampling head 622, thus allowing the filter 33 to be positioned on its respective slide.

A slide loader system 630 includes a magazine 310 supplying fresh slides on at least one slide support belt 300' (two are shown) in a determined pattern. Projections 301' on the slide support belts 300' sequentially remove slides from the slide magazine 310. The spacing and linear arrangement of the slides on the slide support belts 300' correspond to the spacing and linear arrangement of the group of containers 20 in each container support 100'.

A slide unloader system 640 includes another set of slide support belts 304 for collecting the slides once the minelayer has been transferred to the slides. The slide support belts 304 have another set of projections 305 that are more closely spaced than are the projections 301' on the support belts 300'. The sets of belts 300',304 are driven forward and backward at different relative speeds so as to transfer the relatively widely spaced slides on the support belts 300' to the relatively narrow spacing on the support belts 304. The reduced spacing between slides on the support belts 304 provides increased setting time for any fixative that has been applied to facilitate transfer of the monolayer to the slide.

The support belts 304 move the slides to a collection system 307. The collection system 307 may include one or more handling assemblies for positioning the slides in a cassette or other arrangement for subsequent treatments, e.g., staining.

A container unloader system 650 includes another transmission driven by a motor M5 for organizing a plurality of container supports 100'. From the container unloader system 650, the containers 20 are removed either manually or mechanically from the container supports 100'. The container supports 100' are then reused and the containers 20 are forwarded for storage or disposal.

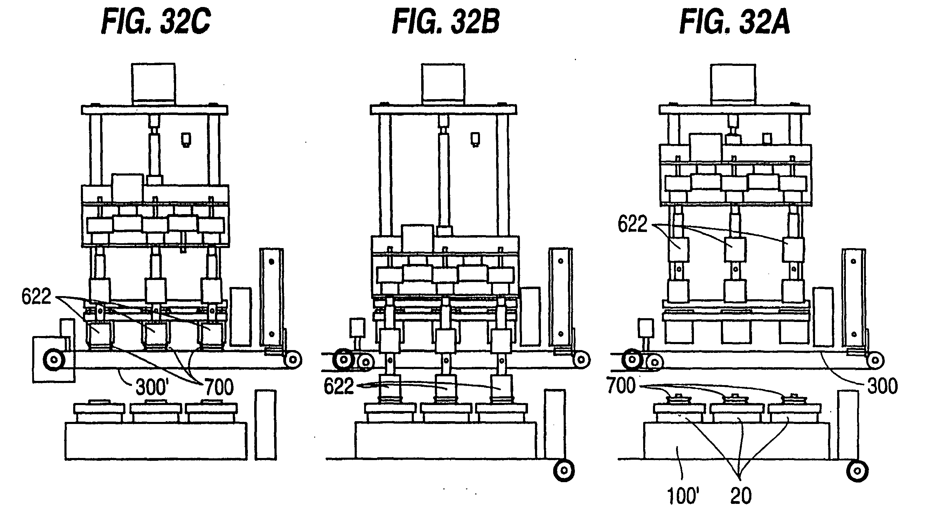

A method of using the second exemplary embodiment is illustrate in Figures 32A-32C. In Figure 32A, a group of containers 20 in a container support 100' have been advanced by the second transmission to the sampling station. The group of sampling heads 622 corresponding to the group of containers 20 are aligned and spaced apart from their respective adapters 700. The slide support belts 300' extend parallel to the groups of containers and slides and are laterally located on opposite sides of the group of adapters 700.

In Figure 32B, the group of sampling heads 622 have been displaced so as to engage their respective adapters 700. A vacuum source is connected to the third port to securely connect each sampling heads 622 to its respective adapter 700. The sample may be mixed by rotating each of the sampling heads 622 relative to its respective container 20. thereby causing further agitation in each sample thus dispersing the particulate matter. A vacuum source is connected to the second ports of each sampling heads 622 to draw at least a portion of the sample in each container 20 through its respective filter 33 thereby collecting a monolayer of particulate matter.

In Figure 32C, the sampling heads 622 are displaced apart from their respective containers 20. Each adapter 700 is securely retained relative to their respective sampling heads 622 by virtue of the vacuum source connected to the third ports of each sampling head 622. The slide support belts 300' are activated to align a respective slide under each of the sampling heads 622. The sampling heads 622 are lowered so as to contact and transfer the monolayer to their respective slide.

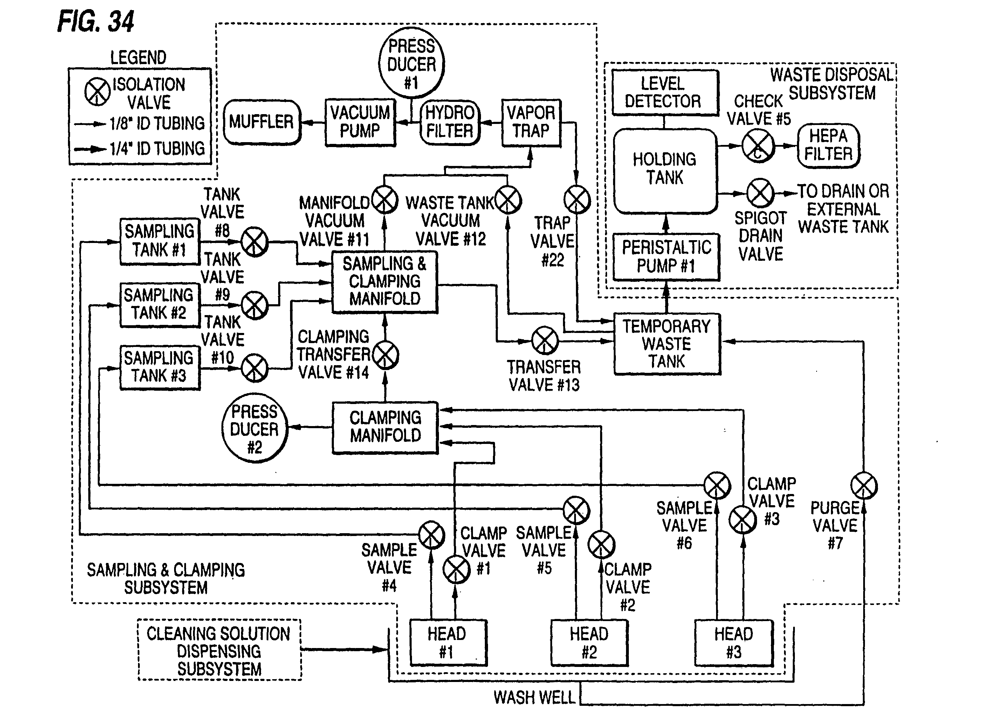

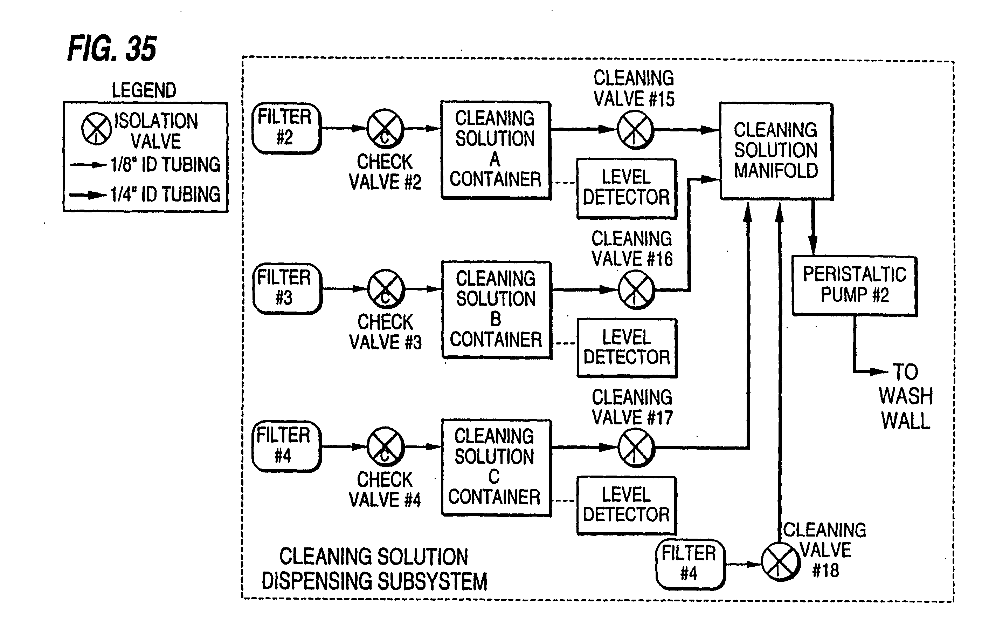

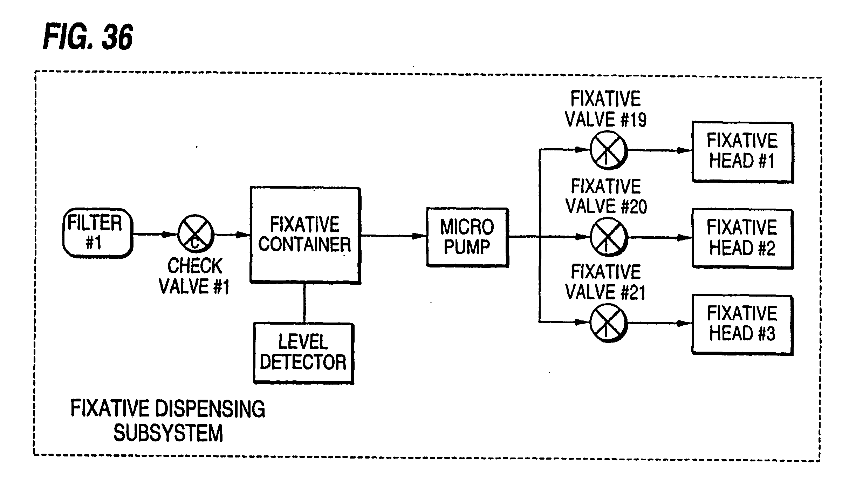

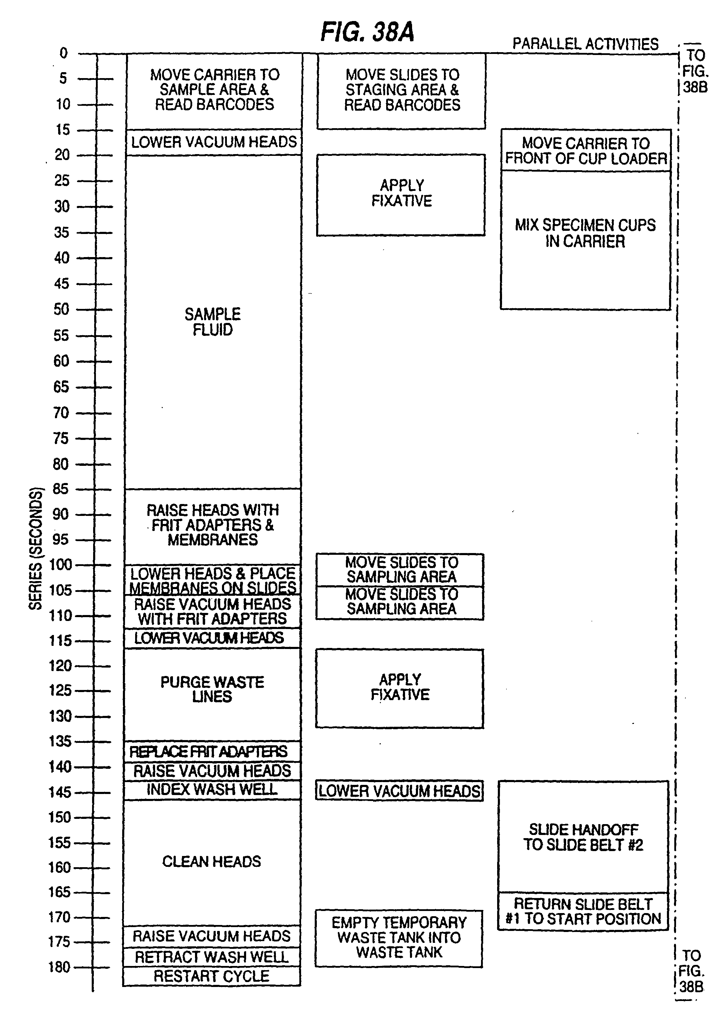

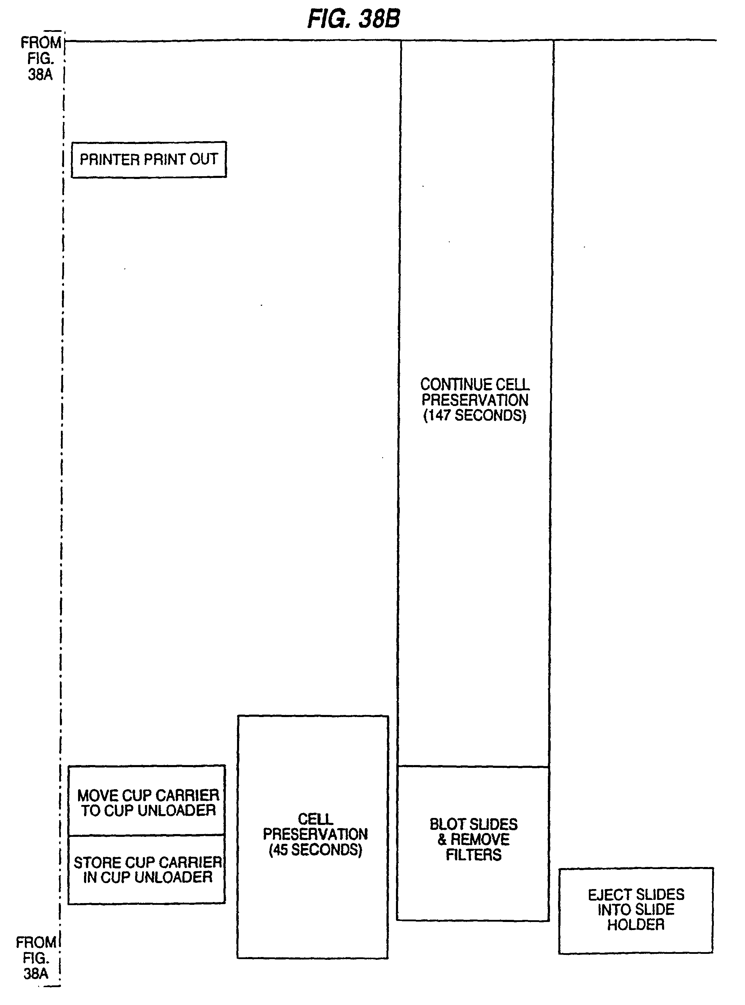

Figures 34-36 show an exemplary fluid flow according to the second preferred embodiment. Figures 37 and 38 show an exemplary arrangement for controlling a process according to the second preferred embodiment.

### ALTERNATIVE CONFIGURATIONS FOR AUTOMATIC PROCESSES

Additionally, a fixative may be applied to the slide before and/or after transfer of the monolayer to the slide. According to a preferred embodiment of the present invention, a fixative dispenser applies at least one drop of a fixative to each of the slides on the support belt 300' prior to the monolayer being transferred to its respective slide. Optionally, a second drop of a fixative may be applied to each of the slides on the support belt 304 after the monolayer has been transferred to its respective slide.

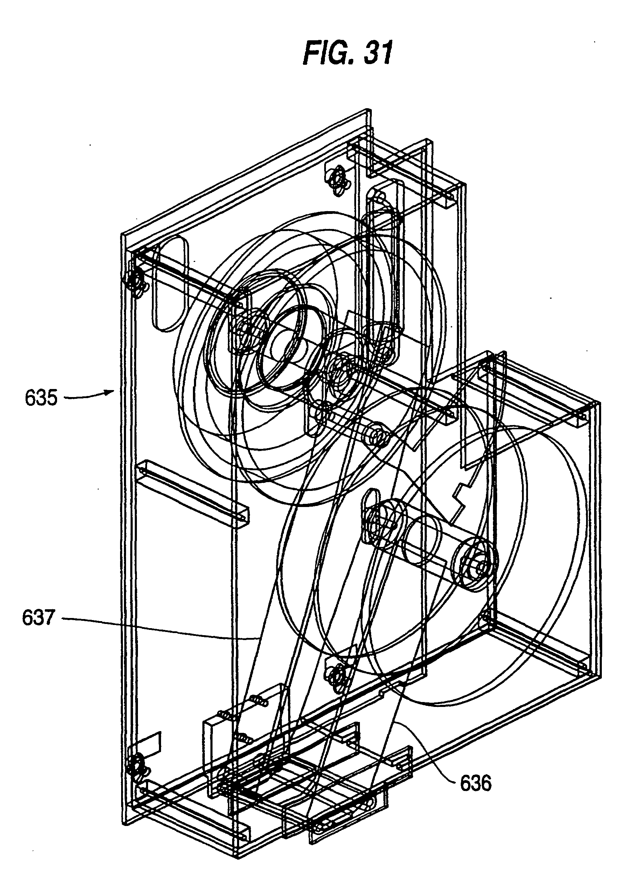

As best seen in Figure 31, a blotter system 635 may be installed preceding the collection system 307. The blotter system 635 blots includes at least one tape supply 636 for absorbing excess fixative. Additionally, a second tape supply 637 may adhesively collect any membranes that remain attached to the monolayer. Of course, both tapes 636,637 are advanced before contacting another slide thus avoiding cross-contamination of the monolayers.

The matter collection apparatus or module described above may be used in combination with other suitable filtration or treatment devices. Exemplary devices include other debris and/or assay devices or modules that may be attached to housing 10. Typically, these additional modules will include a housing having an inlet and an outlet, and will include a filtration, assay, or detection element positioned across the fluid flow path in the housing. For example, the apparatus may comprise a housing including inlet and outlet ports defining a flow path between the inlet and the outlet; a filter positioned across the flow path; and a freely movable chromatography/assay element, such as substrate beads, positioned on the outlet side of the filter. The chromatography/assay element can freely mix with the matter in the fluid, capture the matter, and can then be assayed for the presence of the matter. Suitable devices include those disclosed in U.S. Patents 4,953,561; 5,224,489; 5,016,644; 5,139,031; 5,301,685; 5,042,502; and 5,137,031.

The cytology collection apparatus 10 of the present invention also permits isolation and collection of fresh cells and/or microorganisms from biological fluids to perform DNA probe and chromosomal analysis once the cells arc hemolyzed by the proper buffer.

The most widely used stain for visualization of cellular changes in cytology is the Papanicolaou staining procedure. This stain, which is used for both gynecologic and non-gynecologic applications, is basically composed of blue nuclear and orange, red and green cytoplasmic counterstains. The nuclear stain demonstrates the chromatic patterns associated with normal and abnormal cells, while the cytoplasmic stains help to indicate cell origin. The success of this procedure can be attributed to the ability to observe a number of factors, including definition of nuclear detail and cell differentiation. This staining procedure also results in a multicolor preparation that is aesthetic, possibly reducing eye strain.

Since cellular detail is dependent on fixation, it is preferred that cells be fixed immediately after being deposited on the slide. Too long a delay between preparation and fixation may expose the cells to drying, which may be detrimental to the cellular structure. Moreover, air drying artifacts can adversely affect the subsequent staining results. An exception is when the cells are stained with Wright-Giemsa Staiu, where air drying is used as the fixation step.

In an another embodiment of the present invention, the monolayer of cells may be fixed directly on the collection site. This may be carried out by first depositing a monolayer of cells on the collection site of the cytology collection apparatus as described above and subsequently passing a solution containing a fixative, such as alcohol or acetone, through the cytology collection apparatus.

Included within the scope of the present invention is the production of multiple specimens from a single patient sample. Additional slides for other stain applications can be easily prepared. Human papilloma virus testing, for example, by newer methods such as immunocytochemistry or in-situ hybridization can be performed on the additional slides. As oncogene products or other immunocytochemical tests are developed, more slides may be necessary. The different fixations that these tests may need can easily be incorporated into the procedure since the invention does not require the slides to be fixed in only one way.

This same slide preparation procedure can be used for virtually all forms of cytology. Furthermore, the use of completely contained disposable components addresses biohazard concerns. Ultimately, the enhanced presentation of cells, yielding improved cytologic interpretation, may expand the role of cytology by providing more consistent and reliable patient diagnosis.

Also, captured microorganisms can be cultured in culture medium. After a monolayer of cells has been collected in the cytology collection apparatus, fluid may be used to backflush the collection site, thereby transferring any collected microorganisms from the collection site.

In bacteria testing, the first porous medium can be used for culturing with a Qualture device (not shown) to determine the presence of specific bacteria colonies. The Qualture device is a plastic capsule containing a filter membrane and four nutrient pads of dehydrated, selective media.

The Qualture technique is more sensitive than the agar plate method and more rapid in determining a presumptive diagnosis. The device screens, isolates and presumptively diagnoses bacterial isolates in one step, most often in 4-6 hours. Tests have demonstrated that recovery from fifty milliliters of fluid is excellent and sensitive.

### SEMI-AUTOMATIC APPARATUS

Another apparatus for processing a single sample at a time is illustrated with respect to Figures 39-41. A configuration or structure that engages a portion of the cover or the container typically include any member that positions, fixes and or moves the portion of the cover or the container. Exemplary members include but are not limited to a sleeve, one or more belts, one or more pulleys, one or more resilient bands, and the like.

One embodiment of the present invention includes a support sleeve A for positioning and rotating the container and the outer cover. In a certain embodiment of the invention, the outer portion of the cover also includes one or more resilient bands B that in a loosened or first position (Figure 40) do not engage outer cover 71, and in a tightened or second position (Figure 41) engage and position the outer cover 71 while the outer cover and container are rotating. In an alternative embodiment, belt B may be a drive belt that rotates the outer cover and container around inner cover 72 and tube/agitator 52.

Although the present invention has been described in terms of particular embodiments, it is not limited to those embodiments. Alternative embodiments, examples, and modifications that would still be encompassed by the invention may be made by those skilled in the art, particularly in light of the foregoing teachings.

Additional advantages and modifications will readily occur to those skilled in the art. Therefore, the invention in its broader aspects is not limited to the specific details and representative devices, shown and described herein.

## Claims

1. An automated apparatus (10) for batch processing a set of samples in respective containers (20) for examination, the apparatus comprising:
a container support (100)(100') arranging a group of containers (20) in a first pattern;
a first conveyer (600) advancing said container support with respect to a head stage;
a group of heads (200)(622) corresponding to said group of containers (20) and engaging respective containers at said head stage, each of said group of heads communicating with its respective sample;
a pump producing a flow of each sample from its respective container through its respective head;
a second conveyer (300)(300') arranging a group of slides in a second pattern, said group of slides corresponding to said group of heads, said second conveyer advancing said group of slides to a deposit stage for receiving samples from the group of containers;
a controller coordinating each of said first conveyor advancing said container support, said group of heads engaging said group of containers, said pump producing said flow of each sample, and said second conveyer advancing said group of slides;
wherein said controller coordinates automatic sample throughput and correlating each sample container with a corresponding slide;
**characterised by**:
a group of mixers corresponding to said group of containers (20), each of said group of mixers including an agitator (28)(610) dispersing particulate matter in a respective sample at a mixing stage, the mixing stage being coordinated by the controller; and
a group of filters (33) corresponding to said group of heads, each of said filters communicating with said flow of its respective sample and including:
a membrane (37) being interposed in a first branch (39a) of its respective sample flow and collecting a monolayer of particulate matter; and
a frit (38) being interposed in a second branch (396) of its respective flow circumventing said membrane;
whereby the monolayer of particulate matter from each sample is deposited on a corresponding slide from its respective membrane at the deposit stage.

2. The automated apparatus (10) according to claim 1, wherein said first pattern corresponds to said second pattern.

3. The automated apparatus (10) according to claim 1, wherein said group of mixers further includes a drive rotating each of said heads, and each of said group of containers is fixed with respect to said container support.

4. The automated apparatus (10) according to claim 1, wherein said group of mixers further includes a drive rotating each of said group of containers relative to said container support, and wherein a respective cover for each of said group of containers is prevented from rotating.

5. The automated apparatus (10) according to any one of the preceding claims, wherein each of said agitators (28) (610) stirs its respective sample so as to disperse its respective particulate matter.

6. The automated apparatus (10) according to any one of the preceding claims, further comprising:
a container loader placing said group of containers (20) on said container support (100) (100') in said first pattern;
wherein said controller (400) additionally coordinates said container loader arranging said group of containers on said container support with said first conveyer advancing said container support.

7. The apparatus (10) according to any one of the preceding claims, further comprising:
a container unloader (650) removing said group of containers from said container support at a container unloading stage;
wherein said first conveyer (600) advances said container support (100) (100') from said head stage to said container unloading stage; and
wherein said controller (400) additionally coordinates said container unloader removing said group of containers from said container support with said first conveyer advancing said container support.

8. The automated apparatus (10) according to any one of the preceding claims, further comprising:
a slide loader (70) (630) arranging said group of slides on said second conveyer in said second pattern;
wherein said controller (400) additionally coordinates said slide loader arranging said group of slides on said second conveyer.

9. The automated apparatus (10) according to any one of the preceding claims, further comprising:
a slide unloader (80) (640) removing the slides from said second conveyer at a slide unloading stage;
wherein said second conveyer advances said group of slides from said deposit stage to said slide unloading stage; and
wherein said controller (400) additionally coordinates said slide unloader removing said group of slides from said second conveyer.

10. The automated apparatus (10) according to any one of the preceding claims, further comprising:
a first reader (91) detecting first identifiers on the respective containers;
a second reader (91) detecting second identifiers on the respective slides; and
a printer (90) providing a listing of corresponding first and second identifiers;
wherein said first and second identifiers associate the sample in the respective container with the monolayer of particulate matter on the respective slide; and
wherein said controller (400) additionally coordinates said first and second readers detecting said first and second identifiers and directs said printer marking said corresponding list.

11. The automated apparatus (10) according to any one of the preceding claims, wherein said group of heads reciprocate with respect to said group of containers along a path, and wherein said deposit stage lies along said path.

12. The automated apparatus (10) according to any one of the preceding claims, further comprising:
a plurality of filter sources each supplying a different one of said filters; and
a filter loader (50) transporting each said filter from one of said plurality of filter sources to a filter chamber (30) formed by each of said group of heads engaging its respective container;
wherein said controller (400) additionally coordinates said filter loader transporting said filters from said plurality of filter sources with said group of heads engaging said group of containers.

13. The automated apparatus (10) according to any one of the preceding claims, further comprising:
an applicator supplying a fixative securing the monolayer of particulate matter on the slide;
wherein said controller (400) additionally coordinates said applicator supplying a fixative with said second conveyer advancing said group of slides.

14. The automated apparatus (10) according to claim 13, further comprising:
a blotter (635) absorbing excess fixative;
wherein said controller additionally coordinates said blotter absorbing excess fixative with said applicator supplying a fixative.

15. The automated apparatus (10) according to claim 13, further comprising:
a blower drying said fixative;
wherein said controller additionally coordinates said blower drying said fixative with said applicator supplying a fixative.

16. The automated apparatus (10) according to any one of the preceding claims, further comprising:
a tape separating said membrane from the monolayer of particulate matter on the slide;
wherein said controller additionally coordinates advancing said tape.

17. The automated apparatus (10) according to any one of the preceding claims, further comprising:
a blower separating said membrane from the monolayer of particulate matter on the slide;
wherein said controller additionally coordinates said blower separating said membrane from the monolayer of particulate matter on the slide with said second conveyer advancing said group of slides.

18. The apparatus (10) according to any one of the preceding claims, further comprising:
a solution bath for cleaning said group of heads;
wherein said controller additionally coordinates said solution bath for cleaning said group of heads with said group of heads engaging said group of containers.

19. The apparatus (10) according to any one of the preceding claims, wherein said first and second branches of each said flow pass through said frit for its respective sample.

20. An automated method for batch processing a set of samples in respective containers (20), a monolayer of particulate matter from each sample being deposited on a corresponding slide for examination, the method comprising:
agitating each sample in its respective container;
attaching a group of heads (200) (622) to a corresponding group of sample containers (20), each of said group of heads communicating with its respective sample;
pumping a flow of each sample from its respective container through its respective head;
filtering each said flow with a respective one of a group of filters (33) corresponding to said group of heads, said filtering including:
collecting the monolayer of particulate matter with a membrane (37) in a first branch (39a) of each said flow; and
circumventing said membrane through a frit (38) in a second branch (39b) of each said flow;
transferring each said membrane with its respective monolayer of particulate matter to a respective one of a group of slides, said group of slides corresponding to said group of heads; and
controlling coordination of each of said mixing, attaching, pumping, filtering, and transferring operations to automatically deposit the monolayer of particulate matter from its respective sample to its respective slide.

21. The automated method according to claim 20, further comprising:
concurrently processing new groups of the containers in different ones of said attaching, pumping, filtering, and transferring operations; and
repeating said operations of attaching, pumping, filtering, and transferring for each new group of containers;
wherein said controlling additionally coordinates said concurrent processing of each group of containers with said operations of mixing, attaching, pumping, filtering and transferring.

22. The automated method according to any one of claims 20 and 21, further comprising:
arranging said group of containers on a container support (100) (100') in a first pattern; and
advancing said container support with a first conveyer (600) to said engaging operation;
wherein said controlling additionally coordinates said arranging said group of containers and advancing of said container support (100) (100') with said mixing, attaching, pumping, filtering and transferring operations.

23. The automated method according to any one of claims 20-22, further comprising:
removing said group of containers from said container support (100) (100');
wherein said controlling additionally coordinates said removing of said group of containers with said mixing, attaching, pumping, filtering and transferring operations.

24. The automated method according to any one of claims 20-23, further comprising:
arranging said group of slides on a slide support in a second pattern; and
advancing said group of slides with a second conveyer to said transferring operation;
wherein said controlling additionally coordinates said arranging of said group of slides and advancing of said second conveyer with said mixing, attaching, pumping, filtering, and transferring operations.

25. The automated method according to any one of claims 20-24, further comprising:
removing said group of slides from said slide support;
wherein said controlling additionally coordinates said removing of said group of slides with said mixing, attaching, pumping, filtering and transferring operations.

26. The automated method according to claim 20, wherein said mixing includes rotating each of said heads relative to its respective container.

27. The automated method according to any one of claims 20-26, further comprising:
reading first identifiers on the respective containers; and
printing second identifiers on the respective slides;
wherein said first and second identifiers associate the sample in the respective container with the monolayer of particulate matter on the respective slide; and
wherein said controlling additionally coordinates said reading and printing with said mixing, attaching, pumping, filtering and transferring operations.

28. The automated method according to any one of claims 20-27, further comprising:
selecting said filter from a plurality of filter sources; and
transporting said filter from one of said plurality of filter sources to a filter chamber formed by each of said group of heads engaging its respective container;
wherein said controlling additionally coordinates said selecting and transporting with said mixing, attaching, pumping, filtering and transferring operations.

29. The automated method according to any one of claims 20-28, further comprising:
applying a fixative to the monolayer of particulate matter on its respective slide;
wherein said controlling additionally coordinates said applying with said mixing, attaching, pumping, filtering and transferring operations.

30. The automated method according to claim 29, further comprising:
blotting excess fixative;
wherein said controlling additionally coordinates said blotting with said mixing, attaching, pumping, filtering, transferring and applying operations.

31. The automated method according to claim 29 or 30, further comprising:
drying said fixative with a blower;
wherein said controlling additionally coordinates said drying with said mixing, attaching, pumping, filtering, transferring and applying operations.

32. The automated method according to any one of claims 29-31, further comprising:
separating with a blower said membrane from its respective monolayer of particulate matter on the slide;
wherein said controlling additionally coordinates said separating with said mixing, attaching, pumping, filtering, transferring and applying operations.

33. The automated method according to any one of claims 29-31, further comprising:
separating with a tape said membrane from its respective monolayer of particulate matter on the slide;
wherein said controlling additionally coordinates said separating with said mixing, attaching, pumping, filtering, transferring and applying operations.

34. The automated method according to any one of claims 29-33, further comprising:
cleaning said group of heads (200) (622) in a solution bath;
wherein said controlling additionally coordinates said cleaning with said mixing, attaching, pumping, filtering and transferring operations.

35. The automated method according to any one of claims 29-34, further comprising:
storing the containers (20) having their respective uncollected portions of each sample;
wherein said controlling additionally coordinates said storing with said mixing, attaching, pumping, filtering, transferring and returning operations.

36. The automated method according to any one of claims 29-35, further comprising:
collectively arranging the slides deposited with their respective monolayer of particulate matter;
wherein said controlling additionally coordinates said collectively arranging with said mixing, attaching, pumping, filtering and transferring operations.

## Patentansprüche

1. Automatische Vorrichtung (10) zur Chargenverarbeitung eines Probensatzes in entsprechenden Behältern (20) zur Untersuchung, wobei die Vorrichtung aufweist:
- eine Behälterabstützung (100) (100'), die eine Gruppe Behälter (20) in einem ersten Muster anordnet;
- eine erste Fördereinrichtung (600) für den Transport der Behälterabstützung bezüglich einer Kopfstation;
- eine der Gruppe Behälter (20) entsprechende Gruppe Probenahmeköpfe (200) (622), die mit den entsprechenden Behältern an der Kopfstation in Eingriff kommen,
wobei jeder Kopf der Gruppe Probenahmeköpfe mit seiner jeweiligen Probe verbunden ist;
- eine einen Strom jeder Probe aus ihrem jeweiligen Behälter durch ihren jeweiligen Kopf erzeugenden Pumpe;
- eine zweite Fördereinrichtung (300) (300'), die eine Gruppe Objektträger in einem zweiten Muster anordnet, wobei die Gruppe Objektträger der Gruppe Probenahmeköpfe entspricht, und wobei die zweite Fördereinrichtung die Gruppe Objektträger zu einer Ablagerungsstation für die Aufnahme von Proben aus der Behältergruppe transportiert;
- ein Steuergerät zum Koordinieren der ersten die Behälterabstützung transportierenden Fördereinrichtung, der mit der Behältergruppe in Eingriff stehenden Gruppe Probenahmeköpfe, der den Strom jeder Probe erzeugenden Pumpe und der zweiten die Gruppe Objektträger transportierenden Fördereinrichtung;
wobei das Steuergerät den automatischen Probendurchsatz koordiniert und jeden Probenbehälter mit einem entsprechenden Objektträger korreliert;
**gekennzeichnet durch**:
- eine Mischergruppe entsprechend der Gruppe Behälter (20), wobei jeder Mischer der Mischergruppe einen Rührer (28) (610) enthält, der Teilchen in einer entsprechenden Probe in einer Mischstation dispergiert, wobei die Mischstation vom Steuergerät koordiniert wird; und
- eine der Gruppe Probenahmeköpfe entsprechende Gruppe Filter (33), wobei jeder Filter mit dem Strom seiner jeweiligen Probe verbunden ist und umfasst:
- eine in einen ersten Zweig (39a) des jeweiligen Probenstroms eingesetzte und eine Monoschicht aus Teilchen sammelnde Membran (37); und
- eine in einen die Membran umgehenden zweiten Zweig (396) des jeweiligen Stroms eingesetzte Fritte (38);
wodurch die Teilchen-Monoschicht jeder Probe in der Ablagerungsstation von ihrer entsprechenden Membran auf einem entsprechenden Objektträger abgelagert wird.

2. Automatische Vorrichtung (10) nach Anspruch 1, bei der das erste Muster dem zweiten Muster entspricht.

3. Automatische Vorrichtung (10) nach Anspruch 1, bei der die Mischergruppe ferner einen jeden der Köpfe in Rotation versetzenden Antrieb enthält und jeder Behälter der Behältergruppe bezüglich der Behälterabstützung fixiert ist.

4. Automatische Vorrichtung (10) nach Anspruch 1, bei der die Mischergruppe ferner einen jeden Behälter der Behältergruppe bezüglich der Behälterabstützung in Rotation versetzenden Antrieb enthält und bei der eine entsprechende Abdeckung für jeden Behälter der Behältergruppe an einer Rotation gehindert ist.

5. Automatische Vorrichtung (10) nach einem der vorhergehenden Ansprüche, bei der jeder Rührer (28) (610) seine jeweilige Probe rührt, um die Teilchen zu dispergieren.

6. Automatische Vorrichtung (10) nach einem der vorhergehenden Ansprüche ferner aufweisend:
eine Behälterladeeinrichtung, die die Gruppe Behälter (20) auf der Behälterabstützung (100) (100') im ersten Muster platziert;
wobei das Steuergerät (400) außerdem die Behälterladeeinrichtung bei der Anordnung der Behältergruppe auf der Behälterabstützung mit der die Behälterabstützung transportierenden ersten Fördereinrichtung koordiniert.

7. Automatische Vorrichtung (10) nach einem der vorhergehenden Ansprüche, ferner aufweisend:
eine Behälterentladeeinrichtung (650), die die Behältergruppe von der Behälterabstützung an einer Behälterentladestation entfernt;
wobei die erste Fördereinrichtung (600) die Behälterabstützung (100) (100') von der Kopfstation zur Behälterentladestation transportiert; und
wobei das Steuergerät (400) außerdem die die Behältergruppe von der Behälterabstützung entfernende Behälterentladeeinrichtung mit der die Behälterabstützung transportierenden ersten Fördereinrichtung koordiniert.

8. Automatische Vorrichtung (10) nach einem der vorhergehenden Ansprüche, ferner mit:
einer Objektträger-Ladeeinrichtung (70) (630), die die Gruppe Objektträger auf der zweiten Fördereinrichtung im zweiten Muster anordnet;
wobei das Steuergerät (400) außerdem die die Gruppe Objektträger auf der zweiten Fördereinrichtung anordnende Objektträger-Ladeeinrichtung koordiniert.

9. Automatische Vorrichtung (10) nach einem der vorhergehenden Ansprüche, ferner mit:
einer Objektträger-Entladeeinrichtung (80) (640), die die Objektträger in einer Objektträger-Entladestation von der zweiten Fördereinrichtung entfernt;
wobei die zweite Fördereinrichtung die Gruppe Objektträger von der Ablagerungsstation zur Objektträger-Entladestation transportiert; und
wobei das Steuergerät (400) außerdem die die Gruppe Objektträger von der zweiten Fördereinrichtung entfernende Objektträger-Entladeeinrichtung koordiniert.

10. Automatische Vorrichtung (10) nach einem der vorhergehenden Ansprüche, ferner mit:
einem ersten Lesegerät (91), das erste Identifizierungen auf den jeweiligen Behältern erkennt;
einem zweiten Lesegerät (91), das zweite Identifizierungen auf den jeweiligen Objektträgern erkennt; und
einem Drucker (90), der eine Liste der jeweiligen ersten und zweiten Identifizierungen erstellt;
wobei die ersten und zweiten Identifizierungen die Probe im jeweiligen Behälter der Teilchen-Monoschicht auf dem jeweiligen Objektträger zuordnen; und
wobei das Steuergerät (400) außerdem die die ersten und zweiten Identifizierungen erkennenden Lesegeräte koordiniert und den Drucker anweist, die entsprechende Liste zu markieren.

11. Automatische Vorrichtung (10) nach einem der vorhergehenden Ansprüche, bei der die Gruppe Probenahmeköpfe bezüglich der Gruppe Behälter eine hin- und hergehende Bewegung entlang einer Bahn ausführt, und bei der die Ablagerungsstation auf dieser Bahn liegt.

12. Automatische Vorrichtung (10) nach einem der vorhergehenden Ansprüche, ferner mit:
einer Mehrzahl Filterquellen, von denen eine jede einen anderen Filter beliefert; und
einer Filterladeeinrichtung (50), die jeden Filter aus einer der Mehrzahl Filterquellen zu einer von jedem in Eingriff mit dem jeweiligen Behälter befindlichen Probenahmekopf der Gruppe Probenahmeköpfe gebildeten Filterkammer (30) transportiert;
wobei das Steuergerät (400) außerdem die die Filter von der Mehrzahl Filterquellen transportierende Filterladeeinrichtung mit der mit der Behältergruppe in Eingriff stehenden Gruppe Probenahmeköpfe koordiniert.

13. Automatische Vorrichtung (10) nach einem der vorhergehenden Ansprüche, ferner mit:
einem Applikator, der ein Fixiermittel liefert, das die Teilchen-Monoschicht auf dem Objektträger fixiert;
wobei das Steuergerät (400) außerdem den ein Fixiermittel liefernden Applikator mit der die Gruppe Objektträger transportierenden zweiten Fördereinrichtung koordiniert.

14. Automatische Vorrichtung (10) nach Anspruch 13, ferner mit:
einem Abzieher (635), der überschüssiges Fixiermittel absorbiert;
wobei das Steuergerät außerdem den das überschüssige Fixiermittel absorbierenden Abzieher mit dem ein Fixiermittel liefernden Applikator koordiniert.

15. Automatische Vorrichtung (10) nach Anspruch 13, ferner mit:
einem das Fixiermittel trocknenden Gebläse;
wobei das Steuergerät außerdem das das Fixiermittel trocknende Gebläse mit dem ein Fixiermittel liefernden Applikator koordiniert.

16. Automatische Vorrichtung (10) nach einem der vorhergehenden Ansprüche, ferner mit:
einem Band, das die Membran von der Teilchen-Monoschicht auf dem Objektträger separiert;
wobei das Steuergerät außerdem den Vorschub des Bandes koordiniert.

17. Automatische Vorrichtung (10) nach einem der vorhergehenden Ansprüche, ferner mit:
einem Gebläse, das die Membran von der Teilchen-Monoschicht auf dem Objektträger separiert;
wobei das Steuergerät außerdem das die Membran von der Teilchen-Monoschicht auf dem Objektträger trennende Gebläse mit der die Gruppe Objektträger transportierenden zweiten Fördereinrichtung koordiniert.

18. Vorrichtung (10) nach einem der vorhergehenden Ansprüche, ferner mit:
einem Lösungsbad zum Reinigen der Gruppe Probenahmeköpfe;
wobei das Steuergerät außerdem das Lösungsbad zum Reinigen der Gruppe Probenahmeköpfe mit der mit der Behältergruppe in Eingriff stehenden Gruppe Probenahmeköpfe koordiniert.

19. Vorrichtung (10) nach einem der vorhergehenden Ansprüche, bei der der erste und zweite Zweig jedes Stroms die Fritte für ihre jeweilige Probe passieren.

20. Automatisches Verfahren für die Chargenverarbeitung eines Probensatzes in entsprechenden Behältern (20), bei dem eine Teilchen-Monoschicht von jeder Probe auf einem entsprechenden Objektträger zur Untersuchung abgelagert wird, wobei das Verfahren aufweist:
- Rühren jeder Probe in ihrem jeweiligen Behälter;
- Anbringen einer Gruppe Probenahmeköpfe (200) (622) an einer entsprechenden Gruppe Probenbehälter (20), wobei jeder Kopf der Gruppe Probenahmeköpfe mit seiner entsprechenden Probe verbunden ist;
- Pumpen eines Stroms jeder Probe aus ihrem jeweiligen Behälter durch ihren jeweiligen Kopf;
- Filtrieren jedes Stroms mittels eines entsprechenden Filters einer der Gruppe Probenahmeköpfe entsprechenden Gruppe Filter (33), wobei das Filtrieren beinhaltet:
- Sammeln der Teilchen-Monoschicht mittels einer Membran (37) in einem ersten Zweig (39a) jedes dieser Ströme; und
- Umgehen der Membran durch eine Fritte (38) in einem zweiten Zweig (39b) des Stroms;
- Umsetzen jeder Membran mit ihrer jeweiligen Teilchen-Monoschicht auf einen entsprechenden Objektträger einer Gruppe Objektträger, wobei die Gruppe Objektträger der Gruppe Probenahmeköpfe entspricht; und
- Steuern der Koordination jeder der Misch-, Anbring-, Pump-, Filtrierungs- und Umsetzoperationen, um die Teilchen-Monoschicht von der jeweiligen Probe automatisch auf ihrem entsprechenden Objektträger abzulegen.

21. Automatisches Verfahren nach Anspruch 20, ferner aufweisend:
gleichzeitiges Verarbeiten neuer Behältergruppen in anderen Anbring-, Pump-, Filtrierungs- und Umsetzoperationen; und
Wiederholen der Anbring-, Pump-, Filtrierungs- und Umsetzoperationen für jede neue Behältergruppe;
wobei die Steuerung außerdem die gleichzeitige Verarbeitung jeder Behältergruppe mit den Misch-, Anbring-, Pump-, Filtrierungs- und Umsetzoperationen koordiniert.

22. Automatisches Verfahren nach einem der Ansprüche 20 und 21, ferner aufweisend:
Anordnen der Behältergruppe auf einer Behälterabstützung (100) (100') in einem ersten Muster; und
Transportieren der Behälterabstützung mittels einer ersten Fördereinrichtung (600) zur Eingriffsoperation;
wobei die Steuerung außerdem die Anordnung der Behältergruppe und den Transport der Behälterabstützung (100) (100') mit den Misch-, Anbring-, Pump-, Filtrierungs- und Umsetzoperationen koordiniert.

23. Automatisches Verfahren nach einem der Ansprüche 20 bis 22, ferner aufweisend:
Entfernen der Behältergruppe von der Behälterabstützung (100) (100');
wobei die Steuerung außerdem die Entfernung der Behältergruppe mit den Misch-, Anbring-, Pump-, Filtrierungs- und Umsetzoperationen koordiniert.

24. Automatisches Verfahren nach einem der Ansprüche 20 bis 23, ferner aufweisend:
Anordnen der Gruppe Objektträger auf einer Objektträgerabstützung in einem zweiten Muster; und
Transportieren der Gruppe Objektträger mittels einer zweiten Fördereinrichtung zur Umsetzoperation;
wobei die Steuerung außerdem die Anordnung der Gruppe Objektträger und den Vorschub der zweiten Fördereinrichtung mit den Misch-, Anbring-, Pump-, Filtrierungs- und Umsetzoperationen koordiniert.

25. Automatisches Verfahren nach einem der Ansprüche 20 bis 24, ferner aufweisend:
Entfernen der Gruppe Objektträger von der Objektträgerabstützung;
wobei die Steuerung außerdem die Entfernung der Gruppe Objektträger mit den Misch-, Anbring-, Pump-, Filtrierungs- und Umsetzoperationen koordiniert.

26. Automatisches Verfahren nach Anspruch 20, bei dem das Mischen die Rotation der Köpfe relativ zu ihren jeweiligen Behältern beinhaltet.

27. Automatisches Verfahren nach einem der Ansprüche 20 bis 26, ferner aufweisend:
Lesen erster Identifizierungen auf den jeweiligen Behältern; und
Drucken zweiter Identifizierungen auf die jeweiligen Objektträger;
wobei die ersten und zweiten Identifizierungen die Probe im jeweiligen Behälter der Teilchen-Monoschicht auf dem jeweiligen Objektträger zuordnen; und
wobei die Steuerung außerdem das Lesen und Drucken mit den Misch-, Anbring-, Pump-, Filtrierungs- und Umsetzoperationen koordiniert.

28. Automatisches Verfahren nach einem der Ansprüche 20 bis 27, ferner aufweisend:
Wählen des Filters aus einer Mehrzahl Filterquellen; und
Transportieren des Filters aus einer der Mehrzahl Filterquellen zu einer von jedem mit dem jeweiligen Behälter in Eingriff befindlichen Kopf der Gruppe Probenahmeköpfe gebildeten Filterkammer;
wobei die Steuerung außerdem die Auswahl und den Transport mit den Misch-, Anbring-, Pump-, Filtrierungs- und Umsetzoperationen koordiniert.

29. Automatisches Verfahren nach einem der Ansprüche 20 bis 28, ferner aufweisend:
Aufbringen eines Fixiermittels auf die Teilchen-Monoschicht auf dem jeweiligen Objektträger;
wobei die Steuerung außerdem das Aufbringen mit den Misch-, Anbring-, Pump-, Filtrierungs- und Umsetzoperationen koordiniert.

30. Automatisches Verfahren nach Anspruch 29, ferner aufweisend:
Abziehen des überschüssigen Fixiermittels;
wobei die Steuerung außerdem das Abziehen mit den Misch-, Anbring-, Pump-, Filtrierungs-, Umsetz- und Aufbringoperationen koordiniert.

31. Automatisches Verfahren nach Anspruch 29 oder 30, ferner aufweisend:
Trocknen des Fixiermittels mittels eines Gebläses;
wobei die Steuerung außerdem das Trocknen mit den Misch-, Anbring-, Pump-, Filtrierungs- Umsetz- und Aufbringoperationen koordiniert.

32. Automatisches Verfahren nach einem der Ansprüche 29 bis 31, ferner aufweisend:
Separieren der Membran von ihrer jeweiligen Teilchen-Monoschicht auf dem Objektträger mittels eines Gebläses; träger mittels eines Gebläses;
wobei die Steuerung außerdem das Separieren mit den Misch-, Anbring-, Pump-, Filtrierungs- Umsetz- und Aufbringoperationen koordiniert.

33. Automatisches Verfahren nach einem der Ansprüche 29 bis 31, ferner aufweisend:
Separieren der Membran von ihrer jeweiligen Teilchen-Monoschicht auf dem Objektträger mittels eines Bandes;
wobei die Steuerung außerdem das Separieren mit den Misch-, Anbring-, Pump-, Filtrierungs- Umsetz- und Aufbringoperationen koordiniert.

34. Automatisches Verfahren nach einem der Ansprüche 29 bis 33, ferner aufweisend:
Reinigen der Gruppe Probenahmeköpfe (200) (622) in einem Lösungsbad;
wobei die Steuerung außerdem das Reinigen mit den Misch-, Anbring-, Pump-, Filtrierungs- und Umsetzoperationen koordiniert.

35. Automatisches Verfahren nach einem der Ansprüche 29 bis 34, ferner aufweisend:
Aufbewahren der Behälter (20) mit den jeweiligen nicht gesammelten Anteilen jeder Probe;
wobei die Steuerung außerdem das Aufbewahren mit den Misch-, Anbring-, Pump-, Filtrierungs- Umsetz- und Rückführoperationen koordiniert.

36. Automatisches Verfahren nach einem der Ansprüche 29 bis 35, ferner aufweisend:
kollektives Anordnen der Objektträger mit der darauf abgelagerten jeweiligen Teilchen-Monoschicht;
wobei die Steuerung außerdem das kollektive Anordnen mit den Misch-, Anbring-, Pump-, Filtrierungs- und Umsetzoperationen koordiniert.

## Revendications

1. Appareil automatisé (10) pour le traitement par lots d'une série d'échantillons dans des conteneurs respectifs (20) pour examen, l'appareil comprenant :
un support de conteneurs (100) (100') disposant un groupe de conteneurs (20) dans un premier groupement ;
un premier convoyeur (600) faisant avancer ledit support de conteneurs par rapport à une platine de têtes ;
un groupe de têtes (200) (622) correspondant audit groupe de conteneurs (20) et s'engageant dans des conteneurs respectifs au niveau de ladite platine de têtes, chacune dudit groupe de têtes communiquant avec son échantillon respectif ;
une pompe produisant un écoulement de chaque échantillon depuis son conteneur respectif par sa tête respective ;
un second convoyeur (300) (300') disposant un groupe de lames dans un second groupement, ledit groupe de lames correspondant audit groupe de têtes, ledit second convoyeur faisant avancer ledit groupe de lames vers une platine de dépôt pour recevoir des échantillons du groupe de conteneurs ;
un contrôleur coordonnant chacun dudit premier convoyeur faisant avancer ledit support de conteneurs, dudit groupe de têtes s'engageant dans ledit groupe de conteneurs, de ladite pompe produisant ledit écoulement de chaque échantillon, et dudit second convoyeur faisant avancer ledit groupe
de lames ;
dans lequel ledit contrôleur coordonne un débit d'échantillons automatisé et corrèle chaque conteneur d'échantillon avec une lame correspondante ;
**caractérisé par** :
un groupe de mélangeurs correspondant audit groupe de conteneurs (20), chacun dudit groupe de mélangeurs comportant un agitateur (28) (610) dispersant une matière particulaire dans un échantillon respectif au niveau d'une platine de mélange, la platine de mélange étant coordonnée par le contrôleur ; et
un groupe de filtres (33) correspondant audit groupe de têtes, chacun desdits
filtres communiquant avec ledit écoulement de son échantillon respectif et
comportant :
une membrane (37) étant intercalée dans une première branche (39a) de sa
circulation d'échantillon respective et collectant une couche monomoléculaire de matière particulaire ; et
une fritte (38) étant intercalée dans une seconde branche (396) de sa circulation respective encerclant ladite membrane ;
par lequel la couche monomoléculaire de matière particulaire de chaque échantillon est déposée sur une lame correspondante depuis sa membrane respective au niveau de la platine de dépôt.

2. Appareil automatisé (10) selon la revendication 1, dans lequel ledit premier groupement correspond audit second groupement.

3. Appareil automatisé (10) selon la revendication 1, dans lequel ledit groupe de mélangeurs comporte en outre une commande faisant tourner chacune desdites têtes, et chacun dudit groupe de conteneurs est fixe par rapport audit support de conteneurs.

4. Appareil automatisé (10) selon la revendication 1, dans lequel ledit groupe de mélangeurs comporte en outre une commande faisant tourner chacun dudit groupe de conteneurs par rapport audit support de conteneurs, et dans lequel un couvercle respectif pour chacun dudit groupe de conteneurs est empêché de tourner.

5. Appareil automatisé (10) selon l'une quelconque des revendications précédentes, dans lequel chacun desdits agitateurs (28) (610) agite son échantillon respectif de manière à disperser sa matière particulaire respective.

6. Appareil automatisé (10) selon l'une quelconque des revendications précédentes, comprenant en outre :
un chargeur de conteneurs plaçant ledit groupe de conteneurs (20) sur ledit
support de conteneurs (100) (100') dans leditepremier groupement ;
dans lequel ledit contrôleur (400) coordonne de plus ledit chargeur de conteneurs disposant ledit groupe de conteneurs sur ledit support de conteneurs avec ledit premier convoyeur faisant avancer ledit support de conteneurs.

7. Appareil (10) selon l'une quelconque des revendications précédentes, comprenant en outre :
un déchargeur de conteneurs (650) retirant ledit groupe de conteneurs dudit support de conteneurs au niveau d'une platine de déchargement de conteneurs ;
dans lequel ledit premier convoyeur (600) fait avancer ledit support de conteneurs (100) (100') depuis ladite platine de têtes jusqu'à ladite platine de déchargement de conteneurs ; et
dans lequel ledit contrôleur (400) coordonne de plus ledit déchargeur de conteneurs retirant ledit groupe de conteneurs dudit support de conteneurs avec ledit premier convoyeur faisant avancer ledit support de conteneurs.

8. Appareil automatisé (10) selon l'une quelconque des revendications précédentes, comprenant en outre :
un chargeur de lames (70) (630) disposant ledit groupe de lames sur ledit
second convoyeur dans ledit second groupement ;
dans lequel ledit contrôleur (400) coordonne de plus ledit chargeur de lames disposant ledit groupe de lames sur ledit second convoyeur.

9. Appareil automatisé (10) selon l'une quelconque des revendications précédentes, comprenant en outre :
un déchargeur de lames (80) (640) retirant les lames dudit second convoyeur
au niveau d'une platine de déchargement de lames ;
dans lequel ledit second convoyeur fait avancer ledit groupe de lames depuis ladite platine de dépôt jusqu'à ladite platine de déchargement de lames ; et
dans lequel ledit contrôleur (400) coordonne de plus ledit déchargeur de lames retirant ledit groupe de lames dudit second convoyeur.

10. Appareil automatisé (10) selon l'une quelconque des revendications précédentes, comprenant en outre :
un premier lecteur (91) détectant des premiers identificateurs sur les conteneurs respectifs ;
un second lecteur (91) détectant des seconds identificateurs sur les lames respectives ; et
une imprimante (90) fournissant un listage de premiers et seconds identificateurs correspondants ;
dans lequel lesdits premiers et seconds identificateurs associent l'échantillon dans le conteneur respectif à la couche monomoléculaire de matière particulaire sur la lame respective ; et
dans lequel ledit contrôleur (400) coordonne de plus lesdits premier et second lecteurs détectant lesdits premiers et seconds identificateurs et gère ladite imprimante inscrivant ladite liste correspondante.

11. Appareil automatisé (10) selon l'une quelconque des revendications précédentes, dans lequel ledit groupe de têtes est animé d'un mouvement de va-et-vient par rapport audit groupe de conteneurs le long d'une trajectoire, et dans lequel ladite platine de dépôt se situe le long de ladite trajectoire.

12. Appareil automatisé (10) selon l'une quelconque des revendications précédentes, comprenant en outre :
une pluralité de sources de filtres, chacune fournissant l'un différent desdits filtres ; et
un chargeur de filtres (50) transportant chaque dit filtre depuis l'une de ladite pluralité de sources de filtres jusqu'à une chambre à filtres (30) formée par chacune dudit groupe de têtes s'engageant dans son conteneur respectif ;
dans lequel ledit contrôleur (400) coordonne de plus ledit chargeur de filtres transportant lesdits filtres depuis ladite pluralité de sources de filtres avec ledit groupe de têtes s'engageant dans ledit groupe de conteneurs.

13. Appareil automatisé (10) selon l'une quelconque des revendications précédentes, comprenant en outre :
un applicateur fournissant un fixatif fixant la couche monomoléculaire de matière particulaire sur la lame ;
dans lequel ledit contrôleur (400) coordonne de plus ledit applicateur fournissant un fixatif avec ledit second convoyeur faisant avancer ledit groupe de lames.

14. Appareil automatisé (10) selon la revendication 13, comprenant en outre :
un buvard (635) absorbant l'excès de fixatif ;
dans lequel ledit contrôleur coordonne de plus ledit buvard absorbant l'excès de fixatif avec ledit applicateur fournissant un fixatif.

15. Appareil automatisé (10) selon la revendication 13, comprenant en outre :
un ventilateur séchant ledit fixatif ;
dans lequel ledit contrôleur coordonne de plus ledit ventilateur séchant ledit fixatif avec ledit applicateur fournissant un fixatif.

16. Appareil automatisé (10) selon l'une quelconque des revendications précédentes, comprenant en outre :
une bande séparant ladite membrane de la couche monomoléculaire de matière particulaire sur la lame ;
dans lequel ledit contrôleur coordonne de plus l'avance de ladite bande.

17. Appareil automatisé (10) selon l'une quelconque des revendications précédentes, comprenant en outre :
un ventilateur séparant ladite membrane de la couche monomoléculaire de matière particulaire sur la lame ;
dans lequel ledit contrôleur coordonne de plus ledit ventilateur séparant ladite membrane de la couche monomoléculaire de matière particulaire sur la lame avec ledit second convoyeur faisant avancer ledit groupe de lames.

18. Appareil (10) selon l'une quelconque des revendications précédentes, comprenant en outre :
un bain de solution pour nettoyer ledit groupe de têtes ;
dans lequel ledit contrôleur coordonne de plus ledit bain de solution pour nettoyer ledit groupe de têtes avec ledit groupe de têtes s'engageant dans ledit groupe de conteneurs.

19. Appareil (10) selon l'une quelconque des revendications précédentes, dans lequel lesdites première et seconde branches de chaque dite circulation passent par ladite fritte pour leur échantillon respectif.

20. Procédé automatisé pour le traitement par lots d'une série d'échantillons dans des conteneurs respectifs (20), une couche monomoléculaire de matière particulaire de chaque échantillon étant déposée sur une lame correspondante pour examen, le procédé comprenant :
l'agitation de chaque échantillon dans son conteneur respectif ;
la fixation d'un groupe de têtes (200) (622) à un groupe correspondant de conteneurs d'échantillons (20), chacune dudit groupe de têtes communiquant avec son échantillon respectif ;
le pompage d'un écoulement de chaque échantillon depuis son conteneur respectif par sa tête respective ;
la filtration de chaque dite circulation avec l'un respectif d'un groupe de filtres (33) correspondant audit groupe de têtes, ladite filtration comportant :
la collecte de la couche monomoléculaire de matière particulaire avec une membrane (37) dans une première branche (39a) de chaque dite circulation ;
et le contournement de ladite membrane à travers une fritte (38) dans une seconde branche (39b) de chaque dite circulation ;
le transfert de chaque dite membrane avec sa couche monomoléculaire de matière particulaire respective vers l'une respective d'un groupe de lames, ledit groupe de lames correspondant audit groupe de têtes ; et
la commande de la coordination de chacune desdites opérations de mélange, fixation, pompage, filtration et transfert pour déposer automatisément la couche monomoléculaire de matière particulaire depuis son échantillon respectif sur sa lame respective.

21. Procédé automatisé selon la revendication 20, comprenant en outre :
le traitement simultané de nouveaux groupes des conteneurs dans différentes desdites opérations de fixation, pompage, filtration et transfert ; et
la répétition desdites opérations de fixation, pompage, filtration et transfert pour chaque nouveau groupe de conteneurs ;
dans lequel ladite commande coordonne de plus ledit traitement simultané de chaque groupe de conteneurs avec lesdites opérations de mélange, fixation, pompage, filtration et transfert.

22. Procédé automatisé selon l'une quelconque des revendications 20 et 21, comprenant en outre :
la disposition dudit groupe de conteneurs sur un support de conteneurs (100) (100') dans un premier groupement ; et
l'avance dudit support de conteneurs avec un premier convoyeur (600) jusqu'à ladite opération d'engagement ;
dans lequel ladite commande coordonne de plus lesdites disposition dudit groupe de conteneurs et avance dudit support de conteneurs (100) (100') avec lesdites opérations de mélange, fixation, pompage, filtration et transfert.

23. Procédé automatisé selon l'une quelconque des revendications 20-22, comprenant en outre :
le retrait dudit groupe de conteneurs dudit support de conteneurs (100) (100');
dans lequel ladite commande coordonne de plus ledit retrait dudit groupe de conteneurs avec lesdites opérations de mélange, fixation, pompage, filtration et transfert.

24. Procédé automatisé selon l'une quelconque des revendications 20-23, comprenant en outre :
la disposition dudit groupe de lames sur un support de lames dans un second groupement ; et
l'avance dudit groupe de lames avec un second convoyeur jusqu'à ladite opération de transfert ;
dans lequel ladite commande coordonne de plus lesdites disposition dudit groupe de lames et avance dudit second convoyeur avec lesdites opérations de mélange, fixation, pompage, filtration et transfert.

25. Procédé automatisé selon l'une quelconque des revendications 20-24, comprenant en outre :
le retrait dudit groupe de lames dudit support de lames ;
dans lequel ladite commande coordonne de plus ledit retrait dudit groupe de lames avec lesdites opérations de mélange, fixation, pompage, filtration et transfert.

26. Procédé automatisé selon la revendication 20, dans lequel ledit mélange comporte la rotation de chacune desdites têtes par rapport à son conteneur respectif.

27. Procédé automatisé selon l'une quelconque des revendications 20-26, comprenant en outre :
la lecture de premiers identificateurs sur les conteneurs respectifs ; et
l'impression de seconds identificateurs sur les lames respectives ;
dans lequel lesdits premiers et seconds identificateurs associent l'échantillon dans le conteneur respectif à la couche monomoléculaire de matière particulaire sur la lame respective ; et
dans lequel ladite commande coordonne de plus lesdites lecture et impression avec lesdites opérations de mélange, fixation, pompage, filtration et transfert.

28. Procédé automatisé selon l'une quelconque des revendications 20-27, comprenant en outre :
la sélection dudit filtre à partir d'une pluralité de sources de filtres ; et
le transport dudit filtre depuis l'une de ladite pluralité de sources de filtres jusqu'à une chambre à filtres formée par chacune dudit groupe de têtes s'engageant dans son conteneur respectif ;
dans lequel ladite commande coordonne de plus lesdits sélection et transport avec lesdites opérations de mélange, fixation, pompage, filtration et transfert.

29. Procédé automatisé selon l'une quelconque des revendications 20-28, comprenant en outre :
l'application d'un fixatif à la couche monomoléculaire de matière particulaire sur sa lame respective ;
dans lequel ladite commande coordonne de plus ladite application avec lesdites opérations de mélange, fixation, pompage, filtration et transfert.

30. Procédé automatisé selon la revendication 29, comprenant en outre :
l'absorption au buvard de l'excès de fixatif ;
dans lequel ladite commande coordonne de plus ladite absorption au buvard avec lesdites opérations de mélange, fixation, pompage, filtration, transfert et application.

31. Procédé automatisé selon la revendication 29 ou 30, comprenant en outre :
le séchage dudit fixatif avec un ventilateur ;
dans lequel ladite commande coordonne de plus ledit séchage avec lesdites opérations de mélange, fixation, pompage, filtration, transfert et application.

32. Procédé automatisé selon l'une quelconque des revendications 29-31, comprenant en outre :
la séparation avec un ventilateur de ladite membrane de sa couche monomoléculaire de matière particulaire respective sur la lame ;
dans lequel ladite commande coordonne de plus ladite séparation avec lesdites opérations de mélange, fixation, pompage, filtration, transfert et application.

33. Procédé automatisé selon l'une quelconque des revendications 29-31, comprenant en outre :
la séparation avec une bande de ladite membrane de sa couche monomoléculaire de matière particulaire respective sur la lame ;
dans lequel ladite commande coordonne de plus ladite séparation avec lesdites opérations de mélange, fixation, pompage, filtration, transfert et application.

34. Procédé automatisé selon l'une quelconque des revendications 29-33, comprenant en outre :
le nettoyage dudit groupe de têtes (200) (622) dans un bain de solution ;
dans lequel ladite commande coordonne de plus ledit nettoyage avec lesdites opérations de mélange, fixation, pompage, filtration et transfert.

35. Procédé automatisé selon l'une quelconque des revendications 29-34, comprenant en outre :
le stockage des conteneurs (20) ayant leurs portions non collectées respectives de chaque échantillon ;
dans lequel ladite commande coordonne de plus ledit stockage avec lesdites opérations de mélange, fixation, pompage, filtration; transfert et retour.

36. Procédé automatisé selon l'une quelconque des revendications 29-35, comprenant en outre :
la disposition collective des lames déposées avec leur couche monomoléculaire de matière particulaire respective ;
dans lequel ladite commande coordonne de plus ladite disposition collective avec lesdites opérations de mélange, fixation, pompage, filtration et transfert.
